# EUROPEAN PATENT SPECIFICATION

(11) **EP 2 274 281 B1**
(45) Date of publication and mention of the grant of the patent: **27.05.2015**
(21) Application number: 08874317.4
(22) Date of filing: 04.11.2008
(51) Int. Cl.: C07D 207/16, C07D 207/08, A61P 31/12, C07D 487/04, C07D 519/00, A61K 31/519, A61P 31/14

(54) **HCV PROTEASE INHIBITORS**
HCV-PROTEASE-INHIBITOREN
INHIBITEURS DE PROTÉASE DU VHC

(30) Priority: 16.05.2008 US 53857
(43) Date of publication of application: 19.01.2011
(73) Proprietor: Taigen Biotechnology Co., Ltd., 114 Taipei (TW)
(72) Inventor: LIN, Chu-Chung, Taipei City 104 (TW); LEE, Kuang-Yuan, Hsinchu City 300 (TW); LIU, Yo-Chin, Sanchong City Taipei County 241 (TW); LO, Pin, Sanchong City Taipei County 241 (TW); CHEN, Rong-Jiunn, Chia-I County (TW); LIU, Chen-Fu, Taipei City 114 (TW); CHEN, Chih-Ming, Libertyville IL 60048 (US); KING, Chi-Hsin, Richard, Holladay UT 84124 (US)
(74) Representative: Chajmowicz, Marion
(86) International application number: PCT/US2008/082329
(87) International publication number: WO 2009/139792

(56) References cited:
- WO-A1-2005/028501
- WO-A1-2005/070955
- WO-A2-03/053349
- WO-A2-2004/094452
- WO-A2-2008/022006

## Description

### BACKGROUND

Hepatitis C virus (HCV), a (+)-sense single-stranded RNA virus, is the major causative agent for most cases of non-A, non-B hepatitis. Infection by HCV is a compelling human health problem. See, e.g., WO 05/007681; WO 89/04669; EP 381216; Alberti et al., J. Hepatology, 31 (Suppl. 1), 17-24 (1999); Alter, J. Hepatology, 31 (Suppl. 1), 88-91 (1999); and Lavanchy, J. Viral Hepatitis, 6, 35-47 (1999).

Hepatitis caused by HCV infection is difficult to treat since the virus can quickly mutate and escape the natural immune response. The only anti-HCV therapies currently available are interferon-α, interferon-α/ribavirin combination, and pegylated interferon-α. However, sustained response rates for interferon-α or interferon-α/ribavirin combination were found to be <50% and patients suffer greatly from side effects of these therapeutic agents. See, e.g., Walker, DDT, 4, 518-529 (1999); Weiland, FEMSMicrobial. Rev., 14, 279-288 (1994); and WO 02/18369. Thus, there remains a need for developing more effective and better-tolerated therapeutic drugs.

An HCV protease necessary for viral replication contains about 3000 amino acids. It includes a nucleocapsid protein (C), envelope proteins (E1 and E2), and several non-structural proteins (NS2, NS3, NS4a, NS5a, and NS5b).

NS3 protein possesses serine protease activity and is considered essential for viral replication and infectivity. The essentiality of the NS3 protease was inferred from the fact that mutations in the yellow fever virus NS3 protease decreased viral infectivity. See, e.g., Chamber et al., Proc. Natl. Acad. Sci. USA 87, 8898-8902 (1990). It was also demonstrated that mutations at the active site of the HCV NS3 protease completely inhibited the HCV infection in chimpanzee model. See, e.g., Rice et al., J. Virol. 74 (4) 2046-51 (2000), Further, the HCV NS3 protease was found to facilitate proteolysis at the NS3/NS4a, NS4a/NS4b, NS4b/NS5a, NS5a/NS5b junctions and was thus responsible for generating four viral proteins during viral replication. See, e.g., US 2003/0207861. Consequently, the HCV NS3 protease enzyme is an attractive target in treating HCV infection. Potential NS3 HCV protease inhibitors can be found in WO 02/18369, WO 00/09558, WO 00/09543, WO 99/64442, WO 99/07733, WO 99/07734, WO 99/50230, WO 98/46630, WO 98/17679, WO 97/43310, US 5,990,276, Dunsdon et al., Biorg. Med. Chem. Lett. 10, 1571-1579 (2000); Llinas-Brunet et al., Biorg. Med. Chem. Lett. 10, 2267-2270 (2000); and S. LaPlante et. al., Biorg. Med. Chem. Lett. 10, 2271-2274 (2000).

Certain macrocyclic compounds have been reported to possess HCV inhibiting activity. For example, arylalkoxyl HCV protease inhibitors have been synthesized. See international patent application WO 2008022006. Also certain macrocyclic isoquinoline compounds were found to inhibit HCV. See international patent applications WO 2004094452 and WO 2003053349.

### SUMMARY

This invention is based on the unexpected discovery that certain macrocyclic compounds are effective in inhibiting HCV NS3 activity and HCV RNA levels.

In one aspect, this invention relates to compounds of formula (I): 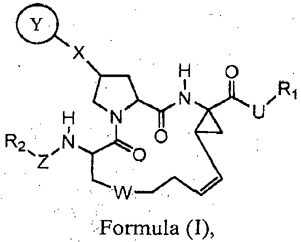 wherein each of R₁ and R₂, independently, is H, C₁₋₆ alkyl, C₁₋₁₀ cycloalkyl, C₁₋₁₀ heterocycloalkyl, aryl, or heteroaryl; U is -O-, -NH-, -NH(CO)-, -NHSO-, or -NHSO₂-; W is -(CH₂)ₘ-, -NH(CH₂)ₙ-, -(CH₂)ₙNH-, -O(CH₂)ₙ-, -(CH₂)ₙO-, -S(CH₂)ₙ-, -(CH₂)ₙS-, -SO-, -SO(CH₂)ₙ-, -(CH₂)ₙSO-, -SO₂(CH₂)ₙ-, or -(CH₂)ₙSO₂-, m being 1,2, or 3 and n being 0, 1, or 2; X is -O-;Y is 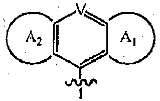 or 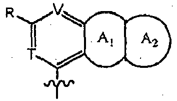 in which each of V and T, independently, is -CH- or -N-; R is H, halo, nitro, cyano, amino, C₁₋₆ alkyl, C₁₋₆ alkoxyl, C₂₋₆ alkenyl, C₂₋₆ alkynyl, C₃₋₁₀ cycloalkyl, C₁₋₁₀ heterocycloalkyl, aryl, or heteroaryl; and each of A₁ and A₂, independently, is C₄₋₁₀ cycloalkyl, C₁₋₁₀ heterocycloalkyl, aryl, or heteroaryl, each of which is optionally substituted with halo, nitro, cyano, C₁₋₆ alkyl, C₁₋₆ alkoxyl, C₂₋₆ alkenyl, C₂₋₆ alkynyl, aryl, or heteroaryl; or optionally fused with another C₃₋₁₀ cycloalkyl, C₁₋₁₀ heterocycloalkyl, aryl, or heteroaryl, each of which is optionally substituted with halo, nitro, cyano, C₁₋₆ alkyl, C₁₋₆ alkoxyl, C₂₋₆ alkenyl, C₂₋₆ alkynyl,
C₃₋₁₀ cycloalkyl, C₁₋₁₀ heterocycloalkyl, aryl, or heteroaryl; and Z is -C(O), -OC(O)-, -NR'C(O)-, -OC(S)-, -NR'-C(S)-, or -OC(NH)-; in which R' is H, C₁₋₆ alkyl, C₃₋₁₀ cycloalkyl, C₁₋₁₀ heterocycloalkyl, aryl, or heteroaryl. The groups assigned to variables U, W, X, and Z are of course bi-valent. Each of the groups is presented above in the same orientation as that in which the variable it is assigned to is presented in the formula. Take for example the group -NHSO- assigned to the variable U, which, as shown in the formula, is interposed between C=O and R₁. The N atom in this -NHSO- group is bonded to C=O and the S atom bonded to R₁.

Referring to formula (I), a subset of the compounds described above are those having one of the following features: R₁ is cyclopropyl; R₂ is C₁₋₅ alkyl or C₃₋₈ cycloalkyl; W is -CH₂CH₂-, -OCH₂-, -SCH₂-, or -SOCH₂-; U is -NHSO₂-; Z is -OC(O)-; X is O; and Y is 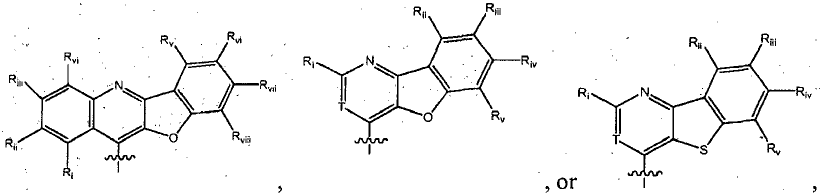 wherein each of Rᵢ, Rᵢᵢ, Rᵢᵢᵢ, Rᵢᵥ, Rᵥ, Rᵥᵢ, Rᵥᵢᵢ, and Rᵥᵢᵢᵢ is, independently, H, halo, nitro, cyano, amino, C₁₋₆ alkyl, C₁₋₆ alkoxyl, C₂₋₆ alkenyl, C₂₋₆ alkynyl, C₃₋₁₀ cycloalkyl, C₁₋₁₀ heterocycloalkyl, aryl, or heteroaryl.

The term "alkyl" refers to a saturated, linear or branched hydrocarbon moiety, such as -CH₃ or -CH(CH₃)₂. The term "alkoxy" refers to an -O-(C₁₋₆ alkyl) radical. The term "alkenyl" refers to a linear or branched hydrocarbon moiety that contains at least one double bond, such as -CH=CH-CH₃. The term "alkynyl" refers to a linear or branched hydrocarbon moiety that contains at least one triple bond, such as -C≡C-CH₃. The term "cycloalkyl" refers to a saturated, cyclic hydrocarbon moiety, such as cyclohexyl. The term "cycloalkenyl" refers to a non-aromatic, cyclic hydrocarbon moiety that contains at least one double bond, such as cyclohexenyl. The term "heterocycloalkyl" refers to a saturated, cyclic moiety having at least one ring heteroatom (e.g., N, O, or S), such as 4-tetrahydropyranyl. The term "heterocycloalkenyl" refers to a non-aromatic, cyclic moiety having at least one ring heteroatom (e.g., N, O, or S) and at least one ring double bond, such as pyranyl. The term "aryl" refers to a hydrocarbon moiety having one or more aromatic rings. Examples of aryl moieties include phenyl (Ph), phenylene, naphthyl, naphthylene, pyrenyl, anthryl, and phenanthryl. The term "heteroaryl" refers to a moiety having one or more aromatic rings that contain at least one heteroatom (e.g., N, O, or S). Examples of heteroaryl moieties include furyl, furylene, fluorenyl, pyrrolyl, thienyl, oxazolyl, imidazolyl, thiazolyl, pyridyl, pyrimidinyl, quinazolinyl, quinolyl, isoquinolyl and indolyl. The term "amino" refers to a radical of -NH₂, -NH-(C₁₋₆ alkyl), or -N(C₁₋₆ alkyl)₂.

Alkyl, alkenyl, alkynyl, cycloalkyl, cycloalkenyl, heterocycloalkyl, heterocycloalkenyl, aryl, and heteroaryl mentioned herein include both substituted and unsubstituted moieties, unless specified otherwise. Possible substituents on cycloalkyl, cycloalkenyl, heterocycloalkyl, heterocycloalkenyl, aryl, and heteroaryl include, but are not limited to, C₁-C₁₀ alkyl, C₂-C₁₀ alkenyl, C₂-C₁₀ alkynyl, C₃-C₂₀ cycloalkyl, C₃-C₂₀ cycloalkenyl, C₁-C₂₀ heterocycloalkyl, C₁-C₂₀ heterocycloalkenyl, C₁-C₁₀ alkoxy, aryl, aryloxy, heteroaryl, heteroaryloxy, amino, C₁-C₁₀ alkylamino, C₁-C₂₀ dialkylamino, arylamino, diarylamino, C₁-C₁₀ alkylsulfonamino, arylsulfonamino, C₁-C₁₀ alkylimino, arylimino, C₁-C₁₀ alkylsulfonimino, arylsulfonimino, hydroxyl, halo, thio, C₁-C₁₀ alkylthio, arylthio, C₁-C₁₀ alkylsulfonyl, arylsulfonyl, acylamino, aminoacyl, aminothioacyl, amidino, guanidine, ureido, cyano, nitro, nitroso, azido, acyl, thioacyl, acyloxy, carboxyl, and carboxylic ester. On the other hand, possible substituents on alkyl, alkenyl, or alkynyl include all of the above-recited substituents except C₁-C₁₀ alkyl. Cycloalkyl, cycloalkenyl, heterocycloalkyl, heterocycloalkenyl, aryl, and heteroaryl can also be fused with each other.

Shown below are exemplary compounds.

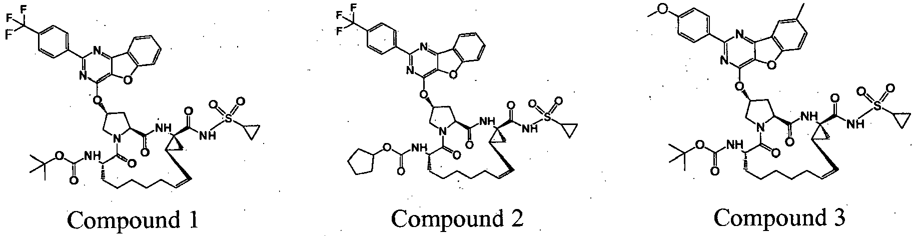

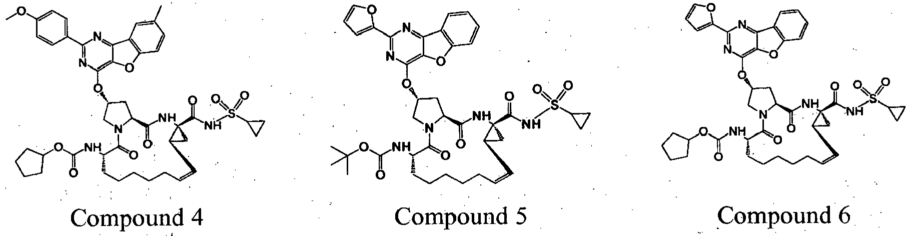

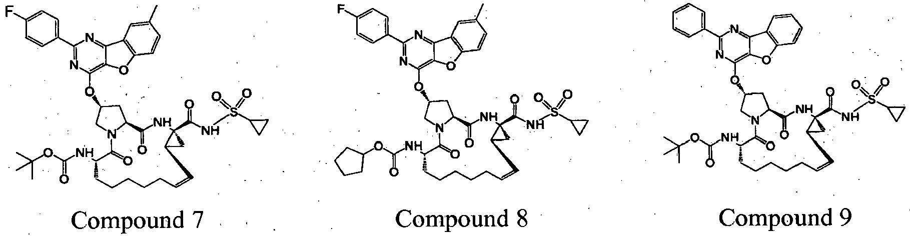

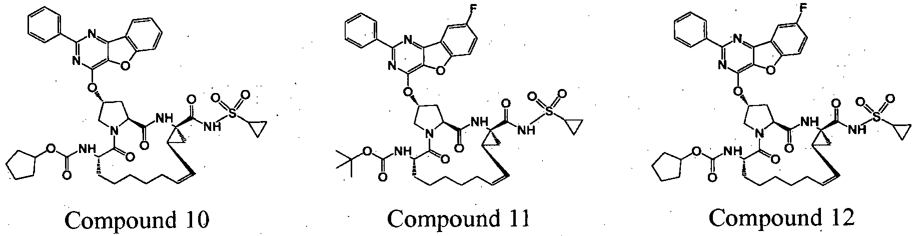

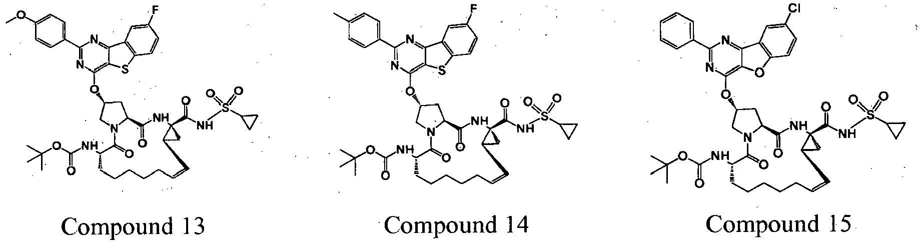

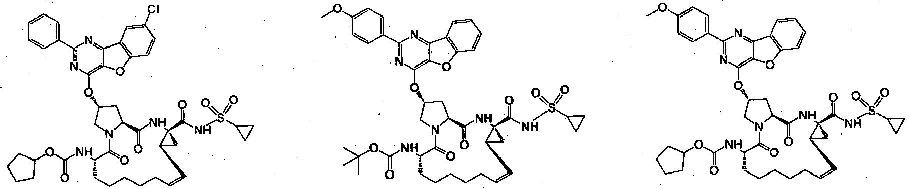

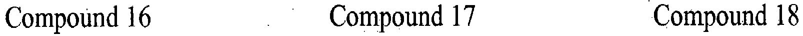

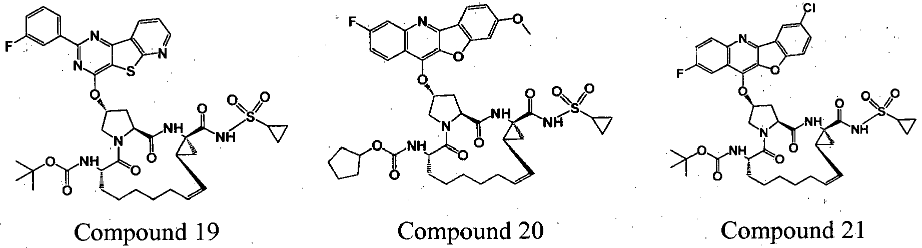

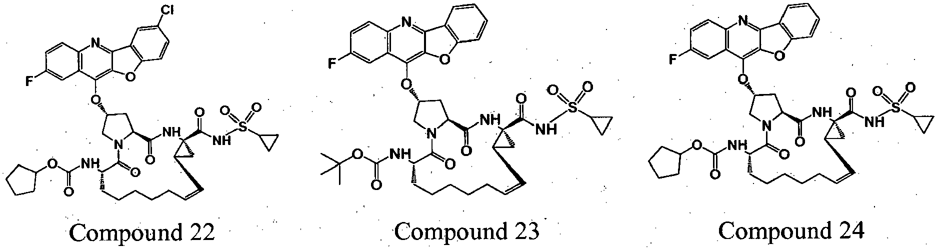

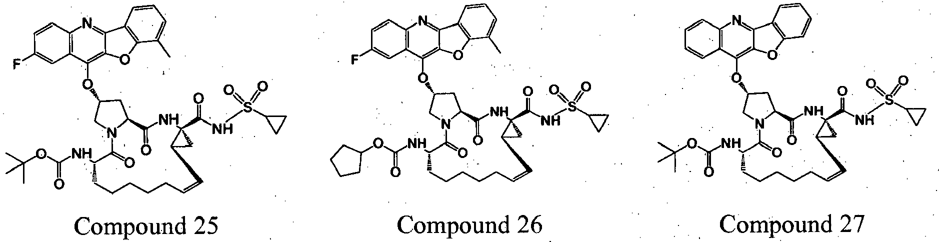

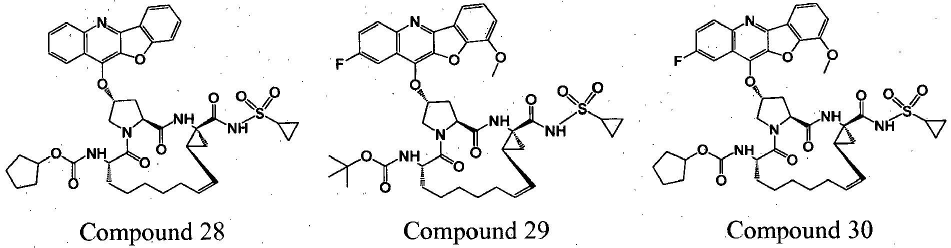

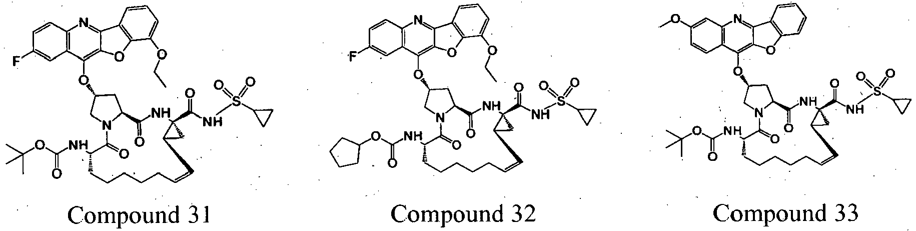

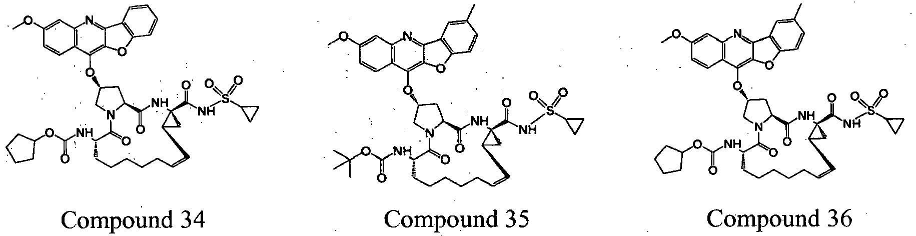

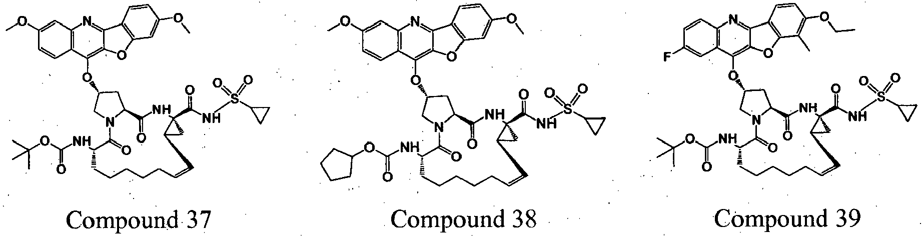

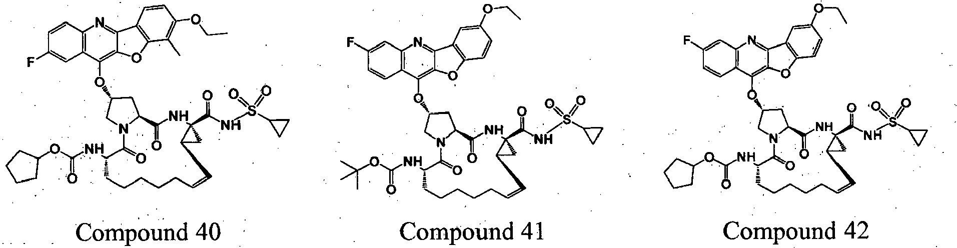

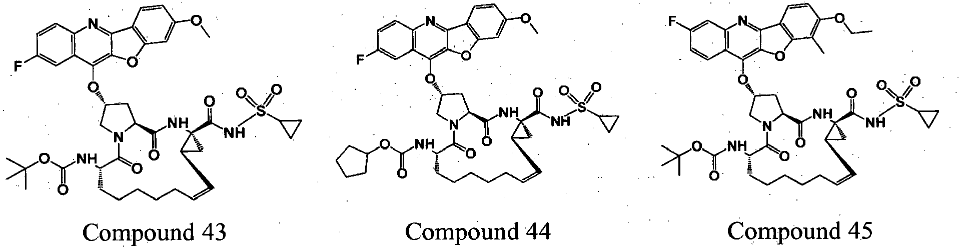

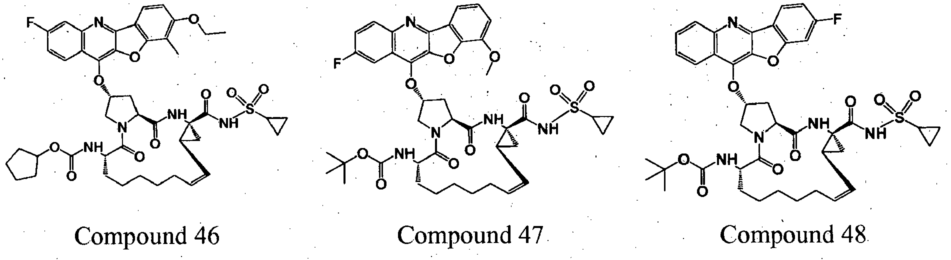

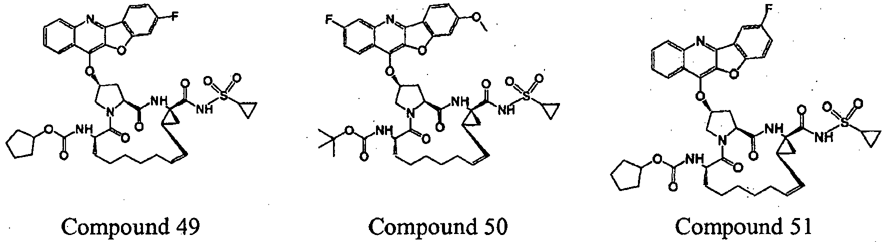

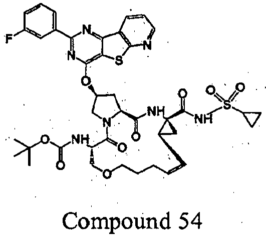

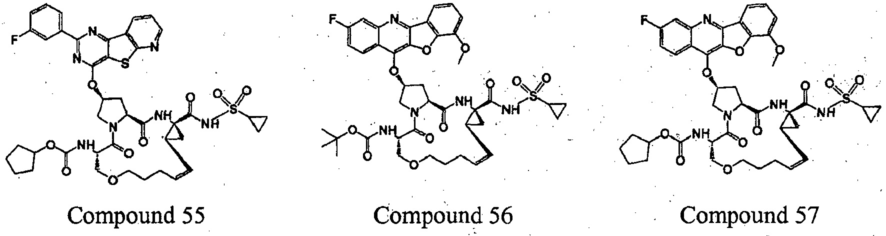

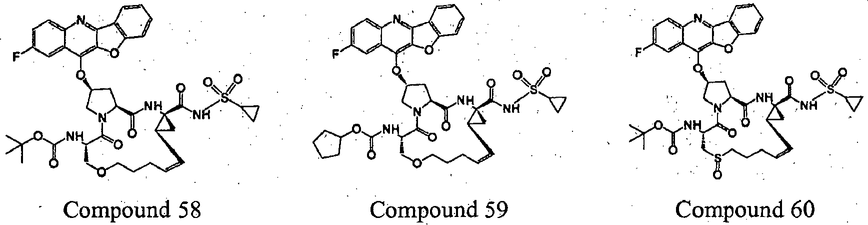

In another aspect, it is described a method for treating hepatitis C virus infection. The method includes administering to a subject in need thereof an effective amount of one or more compounds of formula (I) shown above.

In still another aspect, this invention relates to a pharmaceutical composition for use in treating HCV infection. The composition contains an effective amount of at least one of the compounds of formula (I) and a pharmaceutically acceptable carrier. It may also include an inhibitor of a target other than HCV NS3 protease in the HCV life cycle, e.g., NS5B polymerase, NS5A, NS4B, or p7. Examples of such inhibitors include, but are not limited to, N-[3-(1-cyclobutylmethyl-4-hydroxy-2-oxo-1,2-dihydro-quinolin-3-yl)-1,1-dioxo-1,4-dihydro-116-benzo[1,2,4]thiadiazin-7-yl]-methanesulfonamide (WO04041818), trans-1,2-di-4-[(phenylacetyl-pyrrolidine-2-(S)-carbonyl) amino]-phenylethylene (WO0401413), and 1-aminoadamantane (Amentadine, Griffin, 2004, J. Gen. Virol. 85: p451). The pharmaceutical composition may further contain an immunomodulatory agent or a second antiviral agent. An immunomodulatory agent refers to an active agent that mediates the immune response. Examples of immunomodulatory agents include, but are not limited to, Nov-205 (Novelos Therapeutics Inc., WO02076490) and IMO-2125 (Idera Pharmaceuticals Inc., WO05001055). An antiviral agent refers to an active agent that kills a virus or suppresses its replication. Examples of antiviral agents include, but are not limited to, ribavirin, α-interferon, pegylated interferon, and HCV protease inhibitors, such as 2-(2-{2-cyclohexyl-2-[(pyrazine-2-carbonyl)-amino]-acetylamino}-3,3-dimethyl-butyryl)-octahydro-cyclopenta[c]pyrrole-1-carboxylic acid (1-cyclopropylaminooxalyl-butyl)-amide (Telaprevir, Vertex Pharmaceuticals Inc., WO02018369), 3-[2-(3-tert-butyl-ureido)-3,3-dimethyl-butyryl]-6,6-dimethyl-3-aza-bicyclo[3.1.0]hexane-2-carboxylic acid (2-carbamoyl-1-cyclobutylmethyl-2-oxoethyl)-amide (Boceprevir, Schering-Plough Research Institute, WO03062265), and 4-fluoro-1,3-dihydro-isoindole-2-carboxylic acid 14-tert-butoxycarbonylamino-4-cyclopropanesulfonylaminocarbonyl-2,15-dioxo-3,16-diazatricyclo[14.3.0.04,6]nonadec-7-en-18-yl ester (ITMN-191, InterMune Inc., US2005/0267018).

Also within the scope of this invention is the use of such a composition for the manufacture of a medicament for the just-mentioned treatment.

The details of one or more embodiments of the invention are set forth in the description below. Other features, objects, and advantages of the invention will be apparent from the description and from the claims.

### DETAILED DESCRIPTION

The compounds of this invention can be synthesized from commercially available starting materials by methods well known in the art. For example, one can prepare the compounds of this invention via the route shown in Scheme 1 below:

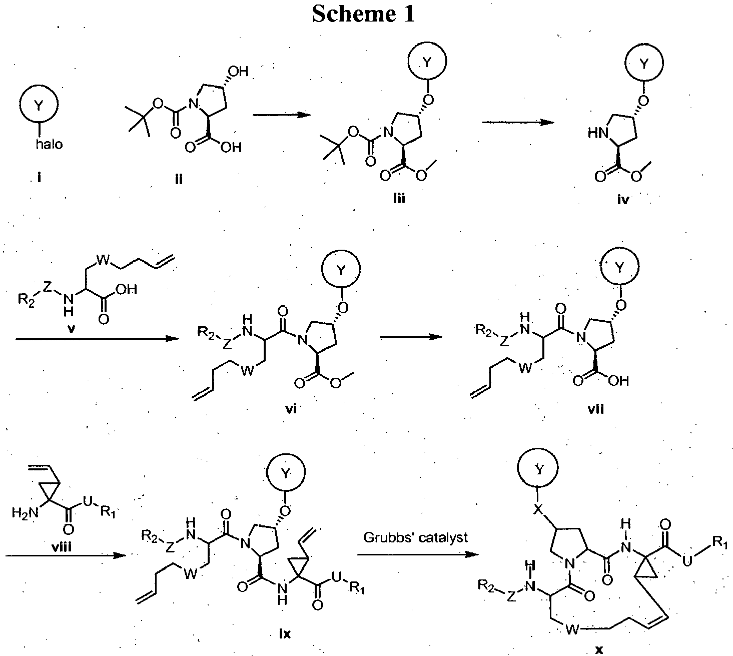

As illustrated in Scheme 1, multicyclic compound **(i)** is first coupled with N-(t-butoxycarbonyl)-L-proline **(ii),** followed by methylation, to form intermediate **(iii)**. Intermediate **(iii)** is deprotected to remove the N-butoxycarbonyl group to produce N-free compound **(iv),** which is coupled with carboxylic acid **(v)** to afford intermediate **(vi).** Intermediate **(vi)** is hydrolyzed to give acid **(vii),** which is coupled with amine compound **(viii)** to provide pyrrolidine compound **(ix)** having two terminal alkenyl groups. Intermediate **(ix)** undergoes olefine metathesis in the presence of Grubbs' catalyst to afford desired macrocyclic compound **(x).**

Schemes 2 and 3 below illustrate two alternative synthetic routes to the compounds of this invention.

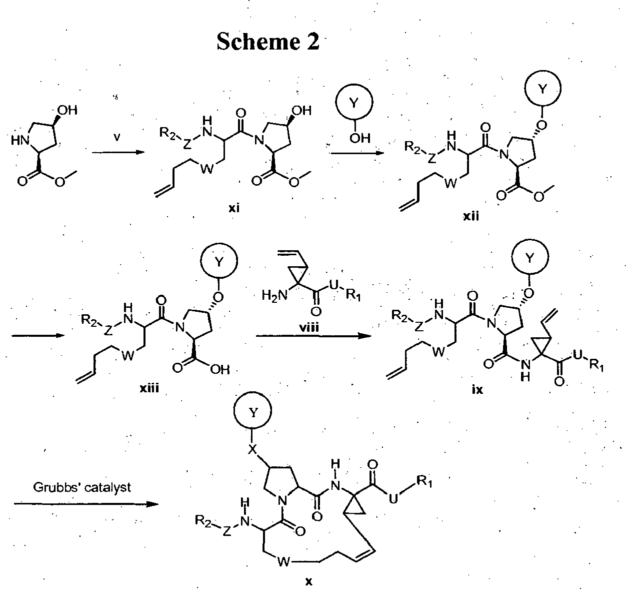

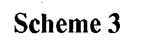

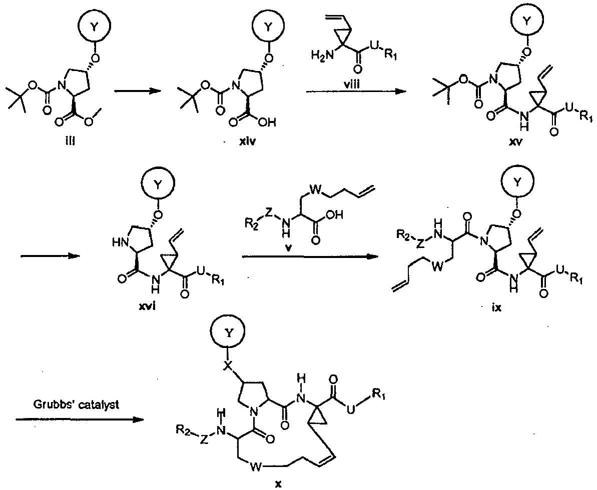

The methods described above may also additionally include steps, either before or after the steps described specifically in Schemes 1-3, to add or remove suitable protecting groups in order to ultimately allow synthesis of the desired compounds. In addition, various synthetic steps may be performed in an alternate sequence or order to give the desired compounds. Synthetic chemistry transformations and protecting group methodologies (protection and deprotection) useful in synthesizing applicable compounds of formula (I) are known in the art and include, for example, those described in R. Larock, Comprehensive Organic Transformations, VCH Publishers (1989); T.W. Greene and P.G.M. Wuts, Protective Groups in Organic Synthesis, 2nd Ed., John Wiley and Sons (1991); L. Fieser and M. Fieser, Fieser and Fieser's Reagents for Organic Synthesis, John Wiley and Sons (1994); and L. Paquette, ed., Encyclopedia of Reagents for Organic Synthesis, John Wiley and Sons (1995) and subsequent editions thereof.

Examples 1-60 below provide detailed descriptions of how exemplary compounds 1-60 were actually prepared.

The compounds mentioned herein contain a non-aromatic double bond and asymmetric centers. Thus, they can occur as racemates and racemic mixtures, single enantiomers, individual diastereomers, diastereomeric mixtures, tautomers, and cis- or trans- isomeric forms. All such isomeric forms are contemplated. For example, the compounds of formula (I) shown above may possess the following stereochemical configuration (III) respectively:

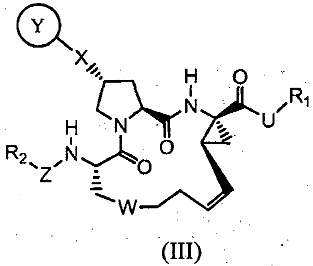

The compounds described above include the compounds themselves, as well as their salts, prodrugs, and solvates, if applicable. A salt, for example, can be formed between an anion and a positively charged group (e.g., amino) on a compound of formula (I). Suitable anions include chloride, bromide, iodide, sulfate, nitrate, phosphate, citrate, methanesulfonate, trifluoroacetate, acetate, malate, tosylate, tartrate, fumurate, glutamate, glucuronate, lactate, glutarate, and maleate. Likewise, a salt can also be formed between a cation and a negatively charged group (e.g., carboxylate) on a compound of formula (I). Suitable cations include sodium ion, potassium ion, magnesium ion, calcium ion, and an ammonium cation such as tetramethylammonium ion. The compounds of formula (I) also include those salts containing quaternary nitrogen atoms. Examples of prodrugs include esters and other pharmaceutically acceptable derivatives, which, upon administration to a subject, are capable of providing active compounds of formula (I). A solvate refers to a complex formed between an active compound of formula (I) and a pharmaceutically acceptable solvent. Examples of pharmaceutically acceptable solvents include water, ethanol, isopropanol, ethyl acetate, acetic acid, and ethanolamine.

Also within the scope of this invention is a method of treating HCV infection by administering an effective amount of one or more of the compounds of formula (I) to a patient. The term "treating" or "treatment" refers to administering the compounds to a subject, who has HCV infection, a symptom of it, or a predisposition toward it, with the purpose to confer a therapeutic effect, e.g., to cure, relieve, alter, affect, ameliorate, or prevent the HCV infection, the symptom of it, or the predisposition toward it. The term "an effective amount" refers to the amount of an active compound of this invention that is required to confer a therapeutic effect on the treated subject. Effective doses will vary, as recognized by those skilled in the art, depending on the types of diseases treated, route of administration, excipient usage, and the possibility of co-usage with other therapeutic treatment.

To practice the method of the present invention, a composition having one or more compounds of this invention can be administered parenterally, orally, nasally, rectally, topically, or buccally. The term "parenteral" as used herein refers to subcutaneous, intracutaneous, intravenous, intrmuscular, intraarticular, intraarterial, intrasynovial, intrasternal, intrathecal, intralesional, or intracranial injection, as well as any suitable infusion technique.

A sterile injectable composition can be a solution or suspension in a non-toxic parenterally acceptable diluent or solvent, such as a solution in 1,3-butanediol. Among the acceptable vehicles and solvents that can be employed are mannitol, water, Ringer's solution, and isotonic sodium chloride solution. In addition, fixed oils are conventionally employed as a solvent or suspending medium (e.g., synthetic mono- or diglycerides). Fatty acid, such as oleic acid and its glyceride derivatives are useful in the preparation of injectables, as are natural pharmaceutically acceptable oils, such as olive oil or castor oil, especially in their polyoxyethylated versions. These oil solutions or suspensions can also contain a long chain alcohol diluent or dispersant, carboxymethyl cellulose, or similar dispersing agents. Other commonly used surfactants such as Tweens or Spans or other similar emulsifying agents or bioavailability enhancers which are commonly used in the manufacture of pharmaceutically acceptable solid, liquid, or other dosage forms can also be used for the purpose of formulation.

A composition for oral administration can be any orally acceptable dosage form including capsules, tablets, emulsions and aqueous suspensions, dispersions, and solutions. In the case of tablets, commonly used carriers include lactose and corn starch. Lubricating agents, such as magnesium stearate, are also typically added. For oral administration in a capsule form, useful diluents include lactose and dried corn starch. When aqueous suspensions or emulsions are administered orally, the active ingredient can be suspended or dissolved in an oily phase combined with emulsifying or suspending agents. If desired, certain sweetening, flavoring, or coloring agents can be added.

A nasal aerosol or inhalation composition can be prepared according to techniques well known in the art of pharmaceutical formulation. For example, such a composition can be prepared as a solution in saline, employing benzyl alcohol or other suitable preservatives, absorption promoters to enhance bioavailability, fluorocarbons, and/or other solubilizing or dispersing agents known in the art.

A composition having one or more active compounds of this invention can also be administered in the form of suppositories for rectal administration.

The carrier in the pharmaceutical composition must be "acceptable" in the sense that it is compatible with the active ingredient of the composition (and preferably, capable of stabilizing the active ingredient) and not deleterious to the subject to be treated. One or more solubilizing agents can be utilized as pharmaceutical excipients for delivery of an active compound of this invention. Examples of other carriers include colloidal silicon oxide, magnesium stearate, cellulose, sodium lauryl sulfate, and D&C Yellow # 10.

The compounds of this invention described above can be preliminarily screened for their efficacy in treating HCV infection by an *in vitro* assay (Examples 61 and 62 below) and then confirmed by animal experiments and clinic trials. Other methods will also be apparent to those of ordinary skill in the art.

The specific examples below are to be construed as merely illustrative, and not limitative of the remainder of the disclosure in any way whatsoever. Without further elaboration, it is believed that one skilled in the art can, based on the description herein, utilize the present invention to its fullest extent. All publications cited herein are hereby incorporated by reference in their entirety.

### Example 1: Synthesis of {4-Cyclopropanesulfonylaminocarbonyl-2,15-dioxo-18-[2-(4-trifluoromethyl-phenyl)-benzo[4,5]furo[3,2-d]pyrimidin-4-yloxy]-3,16-diazatricyclo[14.3.0.04,6]nonadec-7-en-14-yl}-carbamic acid cyclopentyl ester (Compound 1)

Compound **I-3** was first prepared from commercially available 1*-t-*butoxycarbonylamino-2-vinyl-cyclopropanecarboxylic acid ethyl ester via the route shown below:

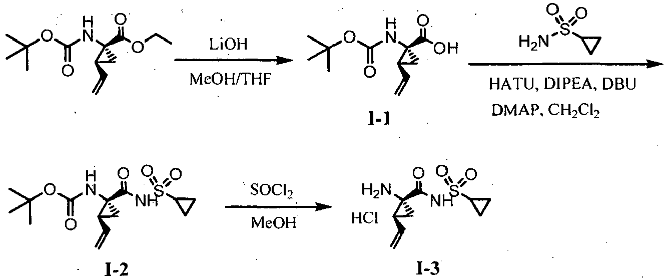

To a solution of 1-*t*-butoxycarbonylamino-2-vinyl-cyclopropanecarboxylic acid ethyl ester (0.34 g, 1.3 mmol) in THF (5 mL) and methanol (5 mL) was added a suspension of LiOH (0.13 g, 5.3 mmol) in water (1.4 mL). After being stirred overnight at room temperature, the reaction was quenched with 10% HCl (2 mL) and the solvent was removed under vacuum. The resultant solid powder was washed with water (10 mL) to give compound **I-1** (0.27 g, 90%). MS m/z 249.9 (M⁺+23); ¹H NMR (CDCl₃) δ 10.35 (brs, 1H), 5.84-5.71 (m, 1H), 5.29 (d, J = 17.4 Hz, 1H), 5.12 (d, J = 10.2 Hz, 1H), 2.23-2.14 (m, 1H), 1.87-1.65 (m, 1H), 1.58-1.41 (m, 1H), 1.43 (s, 9H).

A solution of compound **I-1** (0.52 g, 2.3 mmol), 2-(1H-7-azabenzotriazol-1-yl)-1,1,3,3-tetramethyl uronium hexafluoro-phosphate methanaminium (HATU, 1.74 g, 4.6 mmol), and 4-dimethylaminopyridine (1.39 g, 11.6 mmol) in CH₂Cl₂ (40 mL) was stirred at room temperature for 1 hour, followed by slow addition of cyclopropanesulfonamide (0.57 g, 4.7 mmol), diisopropylethylamine (1.81 mL, 14.0 mmol), and 1,8-diazabicyclo[5,4,0]undec-7-ene (1.80 g, 11.7 mmol) over 15 minutes. After the reaction mixture was stirred at room temperature overnight, the solvent was removed under vacuum. The residue was purified by silica gel column chromatography to give compound **I-2** (0.51 g, 66%). MS m/z 353.1 (M⁺+23); ¹H NMR (CDCl₃) δ 9.75 (brs, 1H), 5.64-5.51 (m, 1H), 5.30 (d, J = 17.4 H), 5.16 (d, J = 10.2 Hz, 1H), 2.95-2.89 (m, 1H), 2.19-2.10 (m, 1H), 1.93-1.88 (m, 1H), 1.47 (s, 9H), 1.46-1.38 (m, 1H), 1.32-1.23 (m, 2H), 1.15-1.00 (m, 2H).

To a solution of compound **1-2** (0.50 g, 1.5 mmol) in MeOH (8 mL) was added SOCl₂ (0.26 g, 2.2 mmol) at room temperature. After the reaction mixture was refluxed for 1 hour, MeOH and SOCl₂ was removed under vacuum. The residue was triturated from pentane and filtered to give intermediate **1-3** as an off-white solid (0.32 g, 91%). MS m/z (M⁺+1); ¹H NMR (CD₃COD) δ 5.77-5.65 (m, 1H), 5.43 (d, J = 17.4 Hz, 1H), 5.32 (d, J = 10.2 Hz, 1H), 3.06-2.97 (m, 1H), 2.45 (dd, J = 17.4 Hz, J = 7.8, 1H), 2.16 (dd, J = 8.0 Hz, J = 7.8 Hz, 1H), 1.75 (dd, J = 10.1 Hz, J = 7.8 Hz, 1H), 1.32-0.86 (m, 4H).

Compound 1 was prepared via the route shown below:

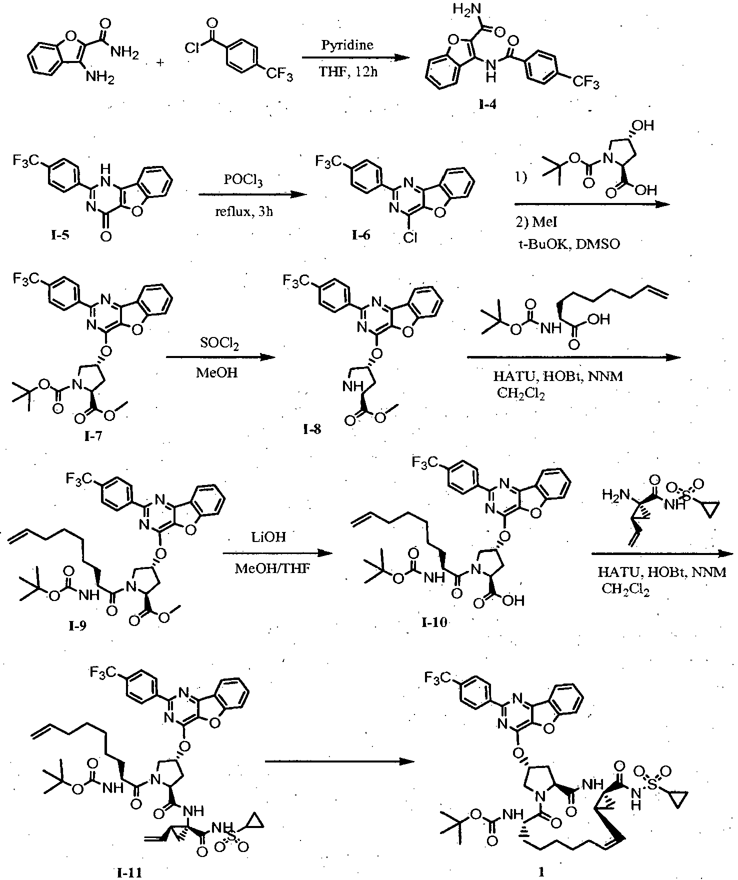

A solution of 3-amino-benzofuran-2-carboxylic acid amide (1.00 g, 5.7 mmol) and pyridine (1 mL, 12.26 mmol) in THF (25 mL) was stirred at 0°C for 10 min. To the resulting solution was slowly added 4-trifluoromethyl-benzoyl chloride (1.48 g, 7.1 mmol). Then the temperature was raised to room temperature and the mixture was stirred for 12 h. After the solvent was removed under reduced pressure, the resulting solid was collected, washed with water, and air-dried to yield **I-4** (1.92 g, 96.0 %). MS: m/z 349.0 (M⁺+1).

To a suspension of **I-4** (1.92 g, 5.5 mmol) and 2N NaOH (13 mL) in EtOH (25 mL) was heated at 85°C for 12h. After cooled, the mixture was acidified and then EtOH was removed. The resulting solid was collected, filtrated, washed with water, and dried to afford **1-5** (1.71 g, 95.0%). MS m/z 331 (M⁺+1).

A solution of **I-5** (1.71 g, 5.2 mmol) and excess phosphorus oxychloride (POCl₃) was refluxed for 2 hours. After cooled and thoroughly concentrated, the mixture was subjected to extraction with methylene chloride and 10% sodium hydroxide. The organic layer was dried over MgSO₄, concentrated, and crystallized from CH₂Cl₂ and *n*-hexane to give compound **I-6** (1.49 g, 82%). MS m/z 348.8, 350.9 (M⁺+¹); ¹H NMR (CDCl₃) δ 8.70 (d, 2H), 8.34 (d, 1H), 7.82-7.75 (m, 4H), 7.57 (ddd, 1H).

To a suspension of boc-trans-4-hydroxy-L-proline (0.53 g, 2.3 mmol) in DMSO (25 mL) was added *t*-BuOK (0.82 g, 5.1 mmol) at 0°C. After the mixture was allowed to warm to room temperature and stirred for 1 hour, compound **I-6** (0.81 g, 2.3 mmol) was added slowly at 10°C. Stirring was continued overnight. Iodomethane (1.02 g, 6.9 mmol) was added and the reaction mixture was stirred at room temperature for additional 30 minutes. The reaction mixture was neutralized to pH 6~7 by 10% HCl aqueous solution and subjected to extraction with methylene chloride. The organic layer was dried over MgSO₄, evaporated under vacuum, and purified by silica gel column chromatography to give compound **I-7** (1.12 g, 86%). MS m/z 557.8 (M⁺+1); ¹H NMR (CDCl₃) δ 8.63 (d, 2H), 8.28 (d, 1H), 7.80-7.74 (m, 2H), 7.70 (d, 2H), 7.51 (ddd, 1H).

To a solution of compound **I**-**7** (1.13 g, 2.0 mmol) in MeOH (20 mL) was added SOCl₂(1.21 g, 9.8 mmol) at room temperature. The reaction mixture was refluxed for 1 hour, and MeOH and SOCl₂ were removed. The residue was triturated in pentane. The suspension was filtered to give compound **I-8** as an off-white solid (0.87 g, 95%). MS m/z 458.1 (M⁺+1).

To a solution of HATU (1.12 g, 3.0 mmol), 1-hydroxybenzotriazole (HOBT, 0.41 g, 3.0 mmol), **I-8** (0.86 g, 1.9 mmol) and 2-*t*-butoxycarbonylamino-non-8-enoic acid (1.21 g, 1.9 mmol) in CH₂Cl₂ (40 mL) at room temperature was added N-methylmorpholine (NMM, 1.02 g, 9.9 mmol). After stirred overnight, the mixture was concentrated under vacuum. The residue was purified by silica gel column chromatography to give compound **I-9** (1.03 g, 73%). MS m/z 711.3 (M⁺+1).

To a solution of compound **I-9** (1.01 g, 1.4 mmol) in THF (20 mL) was added 0.5 M LiOH (5.7 mL, 2.9 mmol) at room temperature. After stirred overnight, the reaction mixture was neutralized by 10% HCl to pH < 7 and concentrated under vacuum. The resultant residue was filtered and washed by water to give compound **I-10** (0.91 g, 92%). MS: m/z 697.3 (M⁺+1).

NMM (0.12 g, 1.2 mmol) was added to a solution of compound **I-3** (0.28 g, 0.4 mmol), HATU(0.31 g, 0.8 mmol), HOBT (0.08 g, 0.6 mmol) and compound **I-10** (0.09 g, 0.4 mmol) in CH₂Cl₂ (10 mL) at room temperature. After stirred overnight, the reaction mixture was concentrated under vacuum. The residue was purified by silica gel column chromatography to give compound **I-11** (0.10 g, 85%). MS m/z 921.3 (M⁺+1); ¹H NMR (CDCl₃) δ 10.24 (s, 1H), 8.61 (d, 2H), 8.26 (d, 1H), 7.77 (d, 2H), 7.73-7.64 (m, 2H), 7.54-7.47 (m, 1H), 7.11 (s, 1H), 6.19 (d, 1H), 5.88-5.70 (m, 2H), 5.38-5.25 (m, 2H), 5.16 (d, 1H), 5.00-4.90 (m, 2H), 4.60 (dd, 1H), 4.88-4.34 (m, 2H), 4.18-4.10 (m, 1H), 2.98-2.89 (m, 1H), 2.68 (dd, 2H), 2.18-1.96 (m, 6H), 1.50-1.32 (m, 7H), 1.28 (s, 9H), 1.09-1.25 (m, 2H):

To a solution of compound **I-11** (0.10 g, 0.11 mmol) in CH₂Cl₂ (10 mL) was added Hoveyda-Grubbs 2^{nd} (35 mg, 0.056 mmol) at room temperature under N₂. Then, the reaction mixture was stirred at 40°C for 24 h to carry out metathesis cyclization. The reaction was quenched and the reaction mixture was purified by column chromatography to give compound 1 (30 mg, 31%). MS: m/z 881.3 (M⁺+1); ¹H NMR (CDCl₃) δ 10.39 (s, 1H), 8.59 (d, 2H), 8.21 (d, 1H), 7.77 (d, 2H), 7.69-7.57 (m, 2H), 7.46 (dd, 1H), 7.20 (s, 1H), 6.12 (s, 1H), 5.69 (q, 1H), 5.12 (d, 1H), 4.97 (dd, 1H), 4.81-4.68 (m, 2H), 4.28-4.07 (m, 2H), 2.96-2.49 (m, 3H), 2.30 (q, 1H), 1.96-1.12 (m, 14H), 1.08 (s, 9H), 0.96-0.82 (m, 2H).

### Example 2: Synthesis of {4-Cyclopropanesulfonylaminocarbonyl-2,15-dioxo-18-[2-(4-trifluoromethyl-phenyl)-benzo[4,5]furo[3,2-d]pyrimidin-4-yloxy]-3,16-diazatricyclo[14.3.0.04,6]nonadec-7-en-14-yl}-carbamic acid cyclopentyl ester (Compound 2)

Compound 2 was prepared via the route shown below:

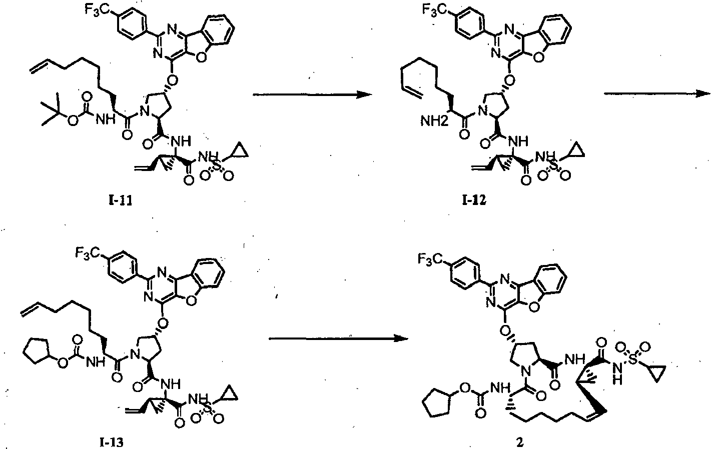

To a solution of compound **I-11** (0.11 g, 0.14 mmol) in 5mL CH₂Cl₂ was added 4N HCl in dioxane (2 mL) at room temperature for 4 hr. HCl, dioxane, and CH₂Cl₂ were removed by evaporation to give crude compound **I-12,** which was used in the next step without further purification.

Crude **I-12** was dissolved in acetonitrile 2 mL and then saturated NaHCO₃ aqueous solution (1 mL) was added. After stirred for 10min, cyclopentyl chloroformate (0.02 g, 0.15 mmol) was added to the reaction mixture at room temperature. The reaction mixture was stirred for another 2 hours. After being quenched by saturated NaHCO₃ aqueous solution, the mixture was subjected to extraction by CH₂Cl₂ to give crude compound **I-13** (0.11 g, 83%). MS: m/z 921.3 (M⁺+1); ¹H NMR (CDCl₃) δ 10.39 (s, 1H), 8.59 (d, 2H), 8.21 (d, 1H), 7.77 (d, 2H), 7.69-7.57 (m, 2H), 7.46 (dd, 1H), 7.20 (s, 1H), 6.12 (s, 1H), 5.69 (q, 1H), 5.12 (d, 1H), 4.97 (dd, 1H), 4.81-4.68 (m, 2H), 4.28-4.07 (m, 2H), 2.96-2.49 (m, 3H), 2.30 (q, 1H), 1.96-1.12 (m, 15H), 1.08 (s, 9H), 0.96-0.82 (m, 2H).

Compound **I-13** was treated with Hoveyda-Grubbs 2^{nd} (35 mg, 0.056 mmol) to carry out metathesis cyclization as described in Example to give compound 2. MS: m/z 893.3 (M⁺+1); ¹H NMR (CDCl₃) δ 10.36 (s, 1H), 8.61 (d, 2H), 8.23 (d, 1H), 7.77 (d, 2H), 7.69-7.43 (m, 3H), 7.09 (s, 1H), 6.16 (s, 1H), 5.71 (q, 1H), 5.17 (d, 1H), 4.98 (dd, 1H), 4.77 (dd, 1H), 4.48 (brs, 1H), 4.63 (d, 1H), 4.30-4.07 (m, 2H), 2.97-2.46 (m, 3H), 2.29 (q, 1H), 1.96-1.06 (m, 22H), 0.96-0.82 (m, 2H).

### Example 3-51: Syntheses of Compound 3-51

Each of Compounds **3-51** was prepared in a manner similar to those described in Examples 1 and 2.

Compound 3: MS: m/z 857.3 (M⁺+1); ¹H NMR (CDCl₃) δ 10.42 (s, 1H), 8.41 (d, 2H), 7.97 (s, 1H), 7.51-7.35 (m, 2H), 7.20 (s, 1H), 7.02 (d, 2H), 6.10 (s, 1H), 5.68 (q, 1H), 5.16 (d, 1H), 4.96 (dd, 1H), 4.75 (dd, 1H), 4.65 (d, 1H), 4.34-4.07 (m, 2H), 3.90 (s, 3H), 2.97-2.50 (m, 3H), 2.51 (s, 3H), 2.30 (q, 1H), 2.05-0.81 (m, 25H).

Compound **4:** MS: m/z 869.3 (M⁺+1);

Compound **5:** MS: m/z 803.3 (M⁺+1); ¹H NMR (CDCl₃) δ 10.3 (s, 1H), 8.29 (d, 1H), 7.67-7.54 (m, 3H), 7.44 (dd, 1H), 7.27 (d, 1H), 7.03 (s, 1H), 6.58 (dd, 1H), 6.09 (s, 1H), 5.69 (q, 1H), 5.05 (d, 1H), 4.97 (dd, 1H), 4.74 (dd, 1H), 4.63 (d, 1H), 4.26-4.04 (m, 2H), 2.95-2.20 (m, 4H), 1.95-1.15 (m, 14H), 1.08 (s, 9H), 0.98-0.81 (m, 2H).

Compound **6:** MS: m/z 815.3 (M⁺+1); ¹H NMR (CDCl₃) δ 10.38 (s, 1H), 8.29 (d, 1H), 7.67-7.38 (m, 4H), 7.28-7.21 (m, 2H), 6.58 (s, 1H), 6.11 (s, 1H), 5.30 (q, 1H), 5.26 (d, 1H), 5.02-4.86 (m, 1H), 4.80-4.68 (m, 1H), 4.57 (d, 1H), 4.53-4.46 (m, 1H), 4.31-4.18 (m, 1H), 4.08 (dd, 1H), 2.96-2.18 (m, 4H), 2.04-0.82 (m, 24H).

Compound **7:** MS: m/z 845.3 (M⁺+1);

Compound **8:** MS: m/z 857.3 (M⁺+1); ¹H NMR (CDCl₃) δ 10.33 (s, 1H), 8.46 (dd, 2H), 7.99 (s, 1H), 7.46 (dd, 2H), 7.18 (dd, 2H), 7.02 (s, 1H), 6.14 (s, 1H), 5.70 (q, 1H), 5.18-4.83 (m, 3H), 4.73 (dd, 1H), 4.55 (d, 1H), 4.36-4.06 (m, 2H), 3.63 (brs, 1H), 2.95-2.46 (m, 3H), 2.51 (s, 3H), 2.25 (q, 1H), 2.05-0.76 (m, 23H).

Compound **9**: MS: m/z 813.3 (M⁺+1); ¹H NMR (CDCl₃) δ 10.44 (s, 1H), 8.48 (dd, 2H), 8.23 (d, 1H), 7.64-7.42 (m, 7H), 6.13 (s, 1H), 5.66 (q, 1H), 5.25 (d, 1H), 4.95 (dd, 1H), 4.75 (dd, 1H), 4.68 (d, 1 H), 4.26-4.10 (m, 2H), 2.96-2.22 (m, 4H), 1.93-1.15 (m, 22H), 1.11 (s, 9H), 0.98-0.80 (m, 2H).

Compound **10:** MS: m/z 825.3 (M⁺+1);

Compound **11:** MS: m/z 831.5 (M⁺+1); ¹H NMR (CDCl₃) δ 10.45 (s, 1H), 8.42 (d, 2H), 7.82 (d, 1H), 7.58-7.42 (m, 4H), 7.40-7.21 (m, 2H), 6.07 (s, 1H), 5.63 (q, 1H), 5.23 (d, 1H), 4.91 (dd, 1H), 4.82-4.70 (m, 1H), 4.67 (d, 1H), 4.24-4.02 (m, 2H), 2.94-2.36 (m, 3H), 2.34-2.18 (m, 1H), 1.94-1.18 (m, 14H), 1.08 (s, 9H), 0.98-0.78 (m, 2H).

Compound **12:** MS: m/z 843.3 (M⁺+1);

Compound **13:** MS: m/z 877.3 (M⁺+1); ¹H NMR (CDCl₃) δ 10.38 (s, 1H), 8.51 (d, 2H), 8.19 (d, 1H), 7.78 (dd, 1H), 7.40-7.24 (m, 2H), 7.06 (d, 2H), 6.12 (s, 1H), 5.70 (q, 1H), 5.10 (d, 1H), 4.98 (dd, 1H), 4.68 (brs, 1H), 4.58 (d, 1H), 4.32-4.12 (m, 2H), 3.91 (s, 3H), 2.98-0.78 (m, 30H).

Compound **14:** MS: m/z 861.3 (M⁺+1); ¹H NMR (CDCl₃) δ 10.35 (s, 1H), 8.43 (d, 2H), 8.18 (dd, 1H), 7.76 (dd, 1H), 7.38-7.28 (m, 3H), 7.08 (s, 1H), 6.11 (s, 1H), 5.69 (q, 1H), 5.02 (d, 1H), 4.95 (dd, 1H), 4.67 (dd, 1H), 4.59 (d, 1H), 4.28-4.08 (m, 2H), 2.95-2.48 (m, 3H), 2.44 (s, 3H), 2.32-2.16 (m, 1H), 1.94-0.78 (m, 25H).

Compound **15:** MS: m/z 847.2, 849.2 (M⁺+1); ¹H NMR (CDCl₃) δ 10.40 (s, 1H), 8.42 (d, 2H), 8.14 (s, 1H), 7.58-7.42 (m, 5H), 7.38 (s, 1H), 6.07 (s, 1H), 5.64 (q, 1H), 5.16 (d, 1H), 4.93 (dd, 1H), 4.75 (dd, 1H), 4.65 (d, 1H), 4.24-4.30 (m, 1H), 2.95-2.20 (m, 4H), 1.96-1.68 (m 5H), 1.60-1.20 (m, 10H), 1.09 (s, 9H), 0.98-0.80 (m, 2H).

Compound **16:** MS: m/z 859.6, 861.6 (M⁺+1); ¹H NMR (CDCl₃) δ 10.29 (s, 1H), 8.46 (dd, 2H), 8.20 (s, 1H), 7.60-7.49 (m, 5H), 6.98 (s, 1H), 6.16 (s, 1H), 5.69 (q, 1H), 5.10 (d, 1H), 4.95 (dd, 1H), 4.73 (dd, 1H), 4.55 (d, 1H), 4.25-4.10 (m, 2H), 2.96-2.22 (m, 4H), 1.96-0.84 (m, 25H).

Compound **17:** MS: m/z 843.3 (M⁺+1); ¹H NMR (CDCl₃) δ 10.41 (s, 1H), 8.44 (d, 2H), 8.23 (d, 1H), 7.68-7.42 (m, 3H), 7.19 (s, 1H), 7.04 (d, 2H), 6.14 (s, 1H), 5.68 (q, 1H), 5.15 (d, 1H), 4.97 (dd, 1H), 4.78-4.73 (m, 1H), 4.65 (d, 1H), 4.25 (dd, 1H), 4.12 (d, 1H), 3.90 (s, 3H), 2.96-2.22 (m, 4H), 1.96-1.17 (m, 14H), 1.13 (s, 9H), 0.96-0.82 (m, 2H).

Compound **18:** MS: m/z 855.3 (M⁺+1); ¹H NMR (CDCl₃) δ 10.37 (s, 1H), 8.45 (d, 2H), 8.25 (d, 1H), 7.70-7.53 (m, 2H), 7.45 (d, 1H), 7.09 (s, 1H), 7.04 (d, 2H), 6.17 (s, 1H), 5.69 (q, 1H), 5.24 (d, 1H), 4.98 (dd, 1H), 4.75 (dd, 1H), 4.57 (d, 1H), 4.30-4.09 (m, 2H), 3.90 (s, 3H), 2.97-2.44 (m, 3H), 2.28 (q, 1H), 1.96-1.06 (m, 25H), 0.96-0.82 (m, 2H).

Compound **19:** MS: m/z 848.3 (M⁺+1); ¹H NMR (CDCl₃) δ 10.48 (s, 1H), 8.77 (s, 1H), 8.74 (d, 1H), 8.34 (d, 1H), 8.24 (d, 1H), 7.55-46 (m, 2H), 7.19 (dd, 1H), 6.96 (d, 1H), 6.14 (s, 1H), 5.70 (q, 1H), 5.05-4.94 (m, 2H), 4.70 (dd, 1H), 4.63 (d, 1H), 4.67-4.51 (m, 2H), 2.96-2.51 (m, 3H), 2.28 (q, 1H), 1.96-1.12 (m, 14H), 1.12 (s, 9H), 0.96-0.82 (m, 2H).

Compound **20:** MS: m/z 846.3 (M⁺+1);

Compound **21:** MS: m/z 838.3 (M⁺+1); ¹H NMR (CDCl₃) δ 10.34 (s, 1H), 8.28 (d, 1H), 8.11 (dd, 1H), 7.82 (dd, 1H), 7.62-7.52 (m, 2H), 7.40 (ddd, 1H), 7.07 (s, 1H), 6.15 (s, 1H), 5.65 (q, 1H), 5.06 (d, 1H), 4.93 (dd, 1H), 4.75 (d, 1H), 4.68 (dd, 1H), 4.26-4.16 (m, 1H), 4.07 (dd, 1H), 2.92-2.50 (m, 3H), 2.30 (q, 1H), 1.93-0.81 (m, 25H).

Compound **22**: MS: m/z 850.3 (M⁺+1); ¹H NMR (CDCl₃) δ 10.34 (s, 1H), 8.28 (d, 1H), 8.10 (dd, 1H), 7.92 (dd, 1H), 7.62-7.52 (m, 2H), 7.38 (ddd, 1H), 7.12 (s, 1H), 6.11 (s, 1H), 5.64 (q, 1H), 5.19 (d, 1H), 4.97-4.83 (m, 2H), 4.76 (d, 1H), 4.66 (dd, 1H), 4.31-4.20 (m, 1H), 4.06 (dd, 1H), 2.94-2.48 (m, 3H), 2.28 (q, 1H), 1.90-0.82 (m, 24H).

Compound **23:** MS: m/z 804.3 (M⁺+1); ¹H NMR (CDCl₃) δ 10.41 (s, 1H), 8.31 (d, 1H), 8.13 (dd, 1H), 7.79 (dd, 1H), 7.67-7.56 (m, 2H), 7.46 (dd, 1H), 7.39 (ddd, 1H), 7.16 (s, 1H), 6.18 (s, 1H), 5.66 (q, 1H), 5.07 (d, 1H), 4.94 (dd, 1H), 4.75 (d, 1H), 4.68 (dd, 1H), 4.27-4.17 (m, 1H), 4.08 (dd, 1H), 2.93-2.48 (m, 3H), 2.31 (q, 1H), 1.92-1.26 (m, 13H), 1.22 (s, 9H), 1.20-0.81 (m, 4H).

Compound **24:** MS: m/z 816.3 (M⁺+1); ¹H NMR (CDCl₃) δ 10.33 (s, 1H), 8.30 (d, 1H), 8.11 (dd, 1H), 7.88 (dd, 1H), 7.67-7.56 (m, 2H), 7.46 (dd, 1H), 7.43-7.30 (m, 2H), 6.12 (s, 1H), 5.64 (q 1H), 5.22 (d, 1H), 4.92 (dd, 1H), 4.77 (d, 1H), 4.66 (dd, 1H), 4.32-4.22 (m, 1H), 4.04 (dd, 1H), 2.93-2.46 (m, 3H), 2.31 (q, 1H), 1.92-0.80 (m, 25H).

Compound **25:** MS: m/z 818.3 (M⁺+1);

Compound **26:** MS: m/z 830.3 (M⁺+1); ¹H NMR (CDCl₃) δ 10.40 (s, 1H), 8.18-8.00 (m, 2H), 7.85 (d, 1H), 7.55 (s, 1H), 7.47-7.25 (m, 3H), 6.09 (s, 1H), 5.65 (q, 1H), 5.21 (d, 1H), 5.02-4.66 (m, 4H), 4.33-4.20 (m, 1H), 4.04 (d, 1H), 4.03 (s, 3H), 2.95-2.40 (m, 6H), 2.32 (q, 1H), 1.96-0.78 (m, 24H).

Compound **27:** MS: m/z 786.3 (M⁺+1);

Compound **28:** MS: m/z 798.3 (M⁺+1); ¹H NMR (CDCl₃) δ 10.37 (s, 1H), 8.35 (d, 1H), 8.22 (d, 1H), 8.17 (d, 1H), 7.67-7.61 (m, 3H), 7.49-7.38 (m, 2H), 7.33 (s, 1H), 6.17 (s, 1H), 5.64 (q, 1H), 5.29 (d, 1H), 4.92 (dd, 1H), 4.83-4.64 (m, 2H), 4.33 (dd, 1H), 4.08 (d, 1H), 2.96-2.24 (m, 4H), 1.91-1.02 (m, 23H), 0.96-0.82 (m, 2H).

Compound **29:** MS: m/z 834.3 (M⁺+1); ¹H NMR (CDCl₃) δ 10.31 (s, 1H), 8.20-8.09 (m, 1H), 7.87 (d, 1H), 7.78 (d, 1H), 7.45-7.32 (m, 2H), 7.14 (s, 1H), 7.12 (d, 1H), 6.25 (s, 1H), 5.66 (q, 1H), 5.06 (d, 1H), 4.94 (dd, 1H), 4.77 (d, 1H), 4.72-4.62 (m, 1H), 4.29-4.14 (m, 1H), 4.09 (d, 1H), 4.03 (s, 3H), 2.93-2.24 (m, 4H), 1.96-0.78 (m, 25H).

Compound **30:** MS: m/z 847.3 (M⁺+1); ¹H NMR (CDCl₃) δ 10.37 (s, 1H), 8.13 (dd, 1H), 7.93-7.77 (m, 2H), 7.46-7.28 (m, 3H), 7.12 (d, 1H), 6.19 (s, 1H), 5.64 (q, 1H), 5.21 (d, 1H), 4.98-4.83 (m, 2H), 4.77 (d, 1H), 4.64 (dd, 1H), 4.34-4.05 (m, 2H), 4.02 (s, 3H), 2.92-2.24 (m, 4H), 1.94-0.76 (m, 24H).

Compound **31:** MS: m/z 848.3 (M⁺+1); ¹H NMR (CDCl₃) δ 10.29 (s, 1H), 8.16 (dd, 1H), 7.88 (d, 1H), 7.81 (dd, 1H), 7.48-7.34 (m, 2H), 7.20-7.06 (d, 2H), 6.27 (s, 1H), 5.69 (q, 1H), 5.05 (d, 1H), 4.96 (dd, 1H), 4.83 (d, 1H), 4.69 (dd, 1H), 4.36-4.16 (m, 3H), 4.10 (dd, 1H), 2.95-2.54 (m, 3H), 2.36 (q, 1H), 1.96-0.81 (m, 28H).

Compound **32:** MS: m/z 860.3 (M⁺+1); ¹H NMR (CDCl₃) δ 10.41 (s, 1H), 8.10 (dd, 1H), 7.93-7.68 (m, 3H), 7.39-7.28 (m, 2H), 7.09 (d, 1H), 6.12 (s, 1H), 5.58 (q, 1H), 5.38 (d, 1H), 4.96-4.76 (m, 3H), 4.68 (dd, 1H), 4.36-4.19 (m, 3H), 4.05 (dd, 1H), 2.92-2.30 (m, 4H), 1.94-0.76 (m, 27H).

Compound **33:** MS: m/z 816.3 (M⁺+1); ¹H NMR (CDCl₃) δ 10.33 (s, 1H), 8.31 (d, 1H), 8.11 (d, 1H), 7.66-7.56 (m, 2H), 7.51-7.41 (m, 2H), 7.06 (s, 1H), 7.05 (dd, 1H), 6.16 (s, 1H), 5.40 (q, 1H), 5.07 (d, 1H), 4.95 (dd, 1H), 4.76 (d, 1H), 4.64 (dd, 1H), 4.32-4.21 (m, 1H), 4.06 (dd, 1H), 3.92 (s, 3H), 2.93-2.24 (m, 4H), 1.94-0.78 (m, 25H).

Compound **34:** MS: m/z 828.3 (M⁺+1); ¹H NMR (CDCl₃) δ 10.41 (s, 1H), 8.30 (d, 1H), 8.08 (d, 1H), 7.67-7.55 (m, 3H), 7.46 (d, 1H), 7.42 (s, 1H), 7.03 (dd, 1H), 6.12 (s, 1H), 5.63 (q, 1H), 5.52-5.40 (m, 1H), 5.32 (d, 1H), 5.10-4.64 (m, 3H), 4.38-4.26 (m, 1H), 4.13-4.02, (m, 1H), 3.90 (s, 3H), 2.93-2.24 (m, 4H), 2.04-0.81 (m, 24H).

Compound **35:** MS: m/z 830.3 (M⁺+1).

Compound **36:** MS: m/z 842.4 (M⁺+1); ¹H NMR (CDCl₃) δ10.37 (s, 1H), 8.11 (s, 1H), 8.09 (d, 1H), 7.54-7.38 (m, 4H), 7.02 (d, 1H), 6.10 (s, 1H), 5.62 (q, 1H), 5.33 (d, 1H), 4.96-4.62 (m, 3H), 4.33 (dd, 1H), 4.06 (dd, 1H), 3.90 (s, 3H), 2.96-2.62 (m, 3H), 2.53 (s, 3H), 2.50-2.24 (m, 1H), 1.91-0.82 (m, 24H).

Compound 37: MS: m/z 846.4 (M⁺+1); ¹HNMR (CDCl₃) δ 10.35 (s, 1H), 8.15 (d, 1H), 8.07 (d, 1H), 7.43 (s, 1H), 7.11-6.96 (m, 4H), 6.09 (s, 1H), 5.68 (q, 1H), 5.10-5.00 (m, 1H), 4.96 (dd, 1H), 4.75 (d, 1H), 4.68-4.57 (m, 1H), 4.34-4.22 (m, 1H), 4.05 (d, 1H), 3.94 (s, 6H), 2.95-2.22 (m, 4H), 1.95-0.76 (m, 25H).

Compound **38:** MS: m/z 858.3 (M⁺+1).

Compound **39:** MS: m/z 862.4 (M⁺+1).

Compound **40:** MS: m/z 874.4 (M⁺+1);

Compound **41:** MS: m/z 848.3 (M⁺+1); ¹H NMR (CDCl₃) δ 10.37 (s, 1H), 8.25 (dd, 1H), 7.75 (dd, 1H), 7.23 (d, 1H), 7.50 (d, 1H), 7.25-7.14 (m, 3H), 6.15 (s, 1H), 5.62 (q, 1H), 5.17 (d, 1H), 4.90 (dd, 1H), 4.76 (d, 1H), 4.68 (dd, 1H), 4.29-4.02 (m, 4H), 2.92-2.45 (m, 3H), 2.29 (q, 1H), 1.92-0.81 (m, 28H).

Compound **42:** MS: m/z 818.3 (M⁺+1); ¹H NMR (CDCl₃) δ 10.34 (s, 1H), 8.26 (dd, 1H), 7.82-7.18 (m, 3H), 7.51 (d, 1H), 7.26-7.14 (m, 2H), 6.18 (s, 1H), 5.66 (q, 1H), 5.21 (d, 1H), 4.94 (dd, 1H), 4.80-4.64 (m, 2H), 4.34-4.02 (m, 5H), 2.92-2.20 (m, 4H), 1.96-0.78 (m, 27H).

Compound **43:** MS: m/z 834.3 (M⁺+1); ¹H NMR (CDCl₃) δ 10.40 (s, 1H), 8.15 (d, 1H), 8.09 (dd, 1H), 7.77 (dd, 1H), 7.43-7.32 (m, 1H), 7.21 (s, 1H), 7.10-7.01 (m, 2H), 6.11 (s, 1H), 5.68 (q, 1H), 5.09 (d, 1H), 4.96 (dd, 1H), 4.76 (d, 1H), 4.68 (dd, 1H), 4.32-4.02 (m, 2H), 3.95 (s, 3H), 2.94-2.28 (m, 4H), 1.96-0.79 (m, 25H).

Compound **44:** MS: m/z 846.3 (M⁺+1); ¹H NMR (CDCl₃) δ 10.34 (s, 1H), 8.18 (d, 1H), 8.12 (dd, 1H), 7.86 (d, 1H), 7.46-7.35 (m, 1H), 7.11 (s, 1H), 7.05 (dd, 1H), 6.98 (s, 1H), 6.12 (s, 1H), 5.71 (q, 1H), 5.12 (d, 1H), 4.02-4.93 (m, 2H), 4.80 (d, 1H), 4.65 (dd, 1H), 4.36-4.24 (m, 1H), 4.12-4.01 (m, 1H), 3.97 (s, 3H), 2.96-2.24 (m, 4H), 1.96-0.79 (m, 24H).

Compound **45:** MS: m/z 862.3 (M⁺+1).

Compound **46:** MS: m/z 874.3 (M⁺+1).

Compound **47:** MS: m/z 834.3 (M⁺+1); ¹H NMR (CDCl₃) δ 10.45 (s, 1H), 8.28 (dd, 1H), 8.07-7.85 (m, 2H), 7.39 (dd, 1H), 7.23-7.13 (m, 3H), 6.40 (s, 1H), 5.66 (q, 1H), 5.02 (d, 1H), 4.93 (dd, 1H), 4.82 (d, 1H), 4.70 (dd, 1H), 4.24-4.14 (m, 1H), 4.09 (dd, 1H), 4.03 (s, 3H), 2.94-2.28 (m, 4H), 1.96-0.79 (m, 25H).

Compound **48:** MS: m/z 804.3 (M⁺+1); ¹H NMR (CDCl₃) δ 10.39 (s, 1H), 8.25 (d, 1H), 8.14 (d, 1H), 8.01 (dd, 1H), 7.65 (dd, 1H), 7.56 (dd, 1H), 7.46-7.31 (m, 3H), 6.14 (s, 1H), 5.62 (q, 1H), 5.21 (d, 1H), 4.90 (dd, 1H), 4.76 (d, 1H), 4.70 (dd, 1H), 4.34-4.23 (m, 1H), 4.14-4.03 (m, 1H), 2.91-2.24 (m, 4H), 2.05-0.82 (m, 25H).

Compound **49:** MS: m/z 816.3 (M⁺+1); ¹H NMR (CDCl₃) δ 10.40 (s, 1H), 8.22 (d, 1H), 8.11 (d, 1H), 7.98 (dd, 1H), 7.62 (dd, 1H), 7.58-7.50 (m, 2H), 7.43-7.28 (m, 2H), 6.10 (s, 1H), 5.57 (q, 1H), 5.36 (d, 1H), 4.96-4.82 (m, 1H), 4.80-4.64 (m, 3H), 4.36-4.01 (m, 2H), 2.91-2.22 (m, 4H), 2.10-0.81 (m, 24H).

Compound **50:** MS: m/z 804.3 (M⁺+1); ¹H NMR (CDCl₃) δ 10.01 (s, 1H), 8.20 (d, 1H), 8.11 (d, 1H), 8.01 (dd, 1H), 7.75 (dd, 1H), 7.58 (d, 1H), 7.35-7.01 (m, 3H), 6.11 (s, 1H), 5.58-5.42 (m, 2H), 4.68 (dd, 1H), 4.19-4.03 (m, 3H), 3.94 (s, 3H), 2.91-2.24 (m, 4H), 2.05-0.82 (m, 25H).

Compound **51:** MS: m/z 816.3 (M⁺+1); ¹H NMR (CDCl₃) δ 10.28 (s, 1H), 8.25 (d, 1H), 8.15 (d, 1H), 8.01 (dd, 1H), 7.67 (dd, 1H), 7.58 (dd, 1H), 7.45 (dd, 1H), 7.35 (ddd, 1H), 6.96 (s, 1H), 6.18 (s, 1H), 5.67 (q, 1H), 5.16 (d, 1H), 4.94 (dd, 1H), 4.98-4.61 (m, 2H), 4.35-4.24 (m, 1H), 4.08 (dd, 1H), 2.91-2.22 (m, 4H), 2.10-0.81 (m, 24H).

### Example 52: Synthesis of {4-Cyclopropanesulfonylamincarbonyl-18-[6-(3-fluorophenyl)-9-thia-1,5,7-triaza-fluoren-8-yloxy]-2,15-dioxo-12-oxa-3,16-diazatricyclo[14.3.0.04,6]nonadec-7-en-14-yl}-carbamic acid tert-butyl ester (Compound 54)

Compound 54 was prepared via the route shown below:

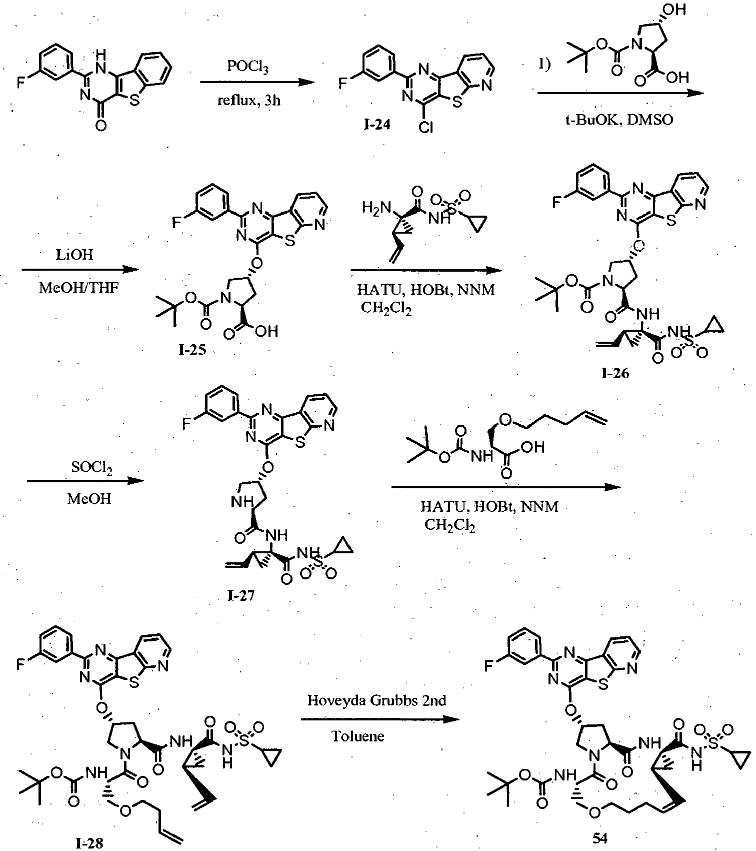

A solution of 6-(3-fluoro-phenyl)-7H-9-thia-1,5,7-triaza-fluoren-8-one (1.83 g, 6.16 mmol) in POCl₃ (5.66 mL, 61.6 mmol) was heated to reflux for 3 hours. After removal of POCl₃ in vacuum, the residue was poured into water and quenched by sat. NaHCO₃ to pH > 7, stirred for 15 minutes, and then filtered to give crude compound **I-24** (1.82 g, 93%). ESI-MS (M+H⁺) = 316.0.

To a suspension of Boc-4R-hydroxyproline (1.33 g, 5.76 mmol) in DMSO (13.3 mL) was added *t*-BuOK (1.94 g, 17.28 mmol) at room temperature. After the reaction mixture was stirred for 1.5 hours, compound **I-24** (1.82 g, 5.76 mmol) was dissolved in DMSO (18.2 mL) and added dropwise to the reaction mixture at ice-water bath for overnight. The resulting mixture was poured into cold water, and the aqueous solution was acidified with 1N HCl to pH < 2 and filtered to give crude compound **1-25** (2.77 g, 94%). ESI-MS (M+H⁺) = 511.1.

NMM (3.67 mL, 33.4 mmol) was added to a solution of compound **I-25** (3.41 g, 6.68 mmol), HATU (5.08 g, 13.36 mmol), HOBt (1.35 g, 10.02 mmol) and cyclopropanesulfonic acid (1-amino-2-vinyl-cyclopropanecarbonyl)-amide (1.69 g, 7.35 mmol) in CH₂Cl₂ (33.4 mL). The reaction mixture was stirred at room temperature for overnight. The resulting mixture was quenched by sat. NH₄Cl, extracted with CH₂Cl₂, washed by sat. NaHCO₃ and brine, dried over MgSO₄, After concentration, the residue was purified by silica gel column chromatography (50 % EtOAc in hexane) to give compound **I-26** (3.20 g, 66%). ESI-MS (M+H⁺) = 723.2.

To a solution of **I**-**26** (0.51 g, 0.69 mmol) in CH₂Cl₂ (3.45 mL) was added CF₃COOH (0.53 mL, 6.9 mmol) at room temperature. After the reaction mixture was stirred overnight, the solution was concentrated to give crude compound **I**-**27**, which was used in the next step without further purification. ESI-MS (M+H⁺) = 623.2.

NMM (0.3 mL, 2.76 mmol) was added to a solution of compound **I-27** (0.43 g, 0.69 mmol), HATU (0.34 g, 0.9 mmol) and (s)-2-(*tert*-butoxycarbonylamino)-3-(pent-4-enyloxy)propanoic acid (0.25 g, 0.9 mmol) in CH₂Cl₂ (3.45 mL). The reaction mixture was stirred at room temperature for overnight. The resulting mixture was quenched by sat. NH₄Cl, extracted with CH₂Cl₂, washed by sat. NaHCO₃ and brine, dried over MSO₄. After concentration, the residue was purified by silica gel column chromatography (50 % EtOAc in hexane) to give compound **I**-**28** (0.52 g, 85%). ESI-MS (M+H⁺) = 877.7

A solution of **1-28** (0.52 g, 0.59 mmol) in toluene (59 mL) was degassed by nitrogen. Hoveyda-Grubbs catalyst 2^{nd} generation (0.04 g, 10% mol) was added at room temperature. The reaction mixture was heated to 50°C for overnight. Concentrated the solvent and the residue was purified by TLC (1 % MeOH in ether) to give compound **54** (0.07 g, 14%). ESI-MS (M+H⁺) = 850.2; ¹H NMR (CDCl₃) δ 10.16 (s, 1H), 8.86-8.58 (m, 2H), 8.38 (d, 1H), 8.27 (d, 1H), 7.57-7.46 (m, 2H), 7.20 (dd, 1H), 6.28-6.20 (m, 1H), 5.64 (q, 1H), 5.31 (d, 1H), 5.21 (dd, 1H), 4.93 (dd, 1H), 4.68-4.58 (m, 1H), 4.39 (dd, 1H), 4.17-4.09 (m, 1H), 3.68 (dd, 1H), 3.50-3.32 (m, 2H), 3.10-2.88 (m, 2H), 2.61-2.46 (m, 2H), 2.26-2.10 (2H), 2.02-1.76 (m, 2H), 1.55-1.38 (m, 4H), 1.34 (s, 9H), 1.31-0.82 (m, 4H).

### Example 53-58: Syntheses of Compound 55-60

Each of Compounds **55-60** was prepared in a manner similar to that described in Example 52.

Compound **55:** MS: m/z 804.3 (M⁺+1); ¹H NMR (CDCl₃) δ 10.19 (s, 1H), 8.81 (d, 1H), 8.37 (d, 1H), 8.27 (d, 1H), 7.60-7.16 (m, 5H), 6.25 (s, 1H), 5.65 (q, 1H), 5.45 (d, 1H), 5.21 (dd, 1H), 4.93 (brs, 1H), 4.74-4.61 (m, 1H), 4.43-4.34 (m, 1H), 4.13 (d, 1H), 3.75-3.34 (m, 4H), 3.08-2.88 (m, 2H), 2.63-2.45 (m, 2H), 2.27-0.81 (m, 19H).

Compound **56:** MS: m/z 804.3 (M⁺+1); ¹H NMR (CDCl₃) δ 10.06 (s, 1H), 8.21 (dd, 1H), 7.91 (d, 1H), 7.81 (dd, 1H), 7.39 (dd, 1H), 7.30-7.22 (m, 1H), 7.16 (d, 1H), 6.24 (s, 1H), 5.63 (q, 1H), 5.39 (d, 1H), 5.18 (dd, 1H), 4.89 (dd, 1H), 4.68-4.58 (m, 1H), 4.43 (d, 1H), 4.13-4.06 (m, 1H), 4.07 (s, 3H), 3.72 (dd, 1H), 3.52 (dd, 1H), 3.45-3.38 (m, 1H), 3.09-3.87 (m, 2H), 2.68 (dd, 1H), 2.53-1.91 (m, 4H), 1.57-1.41 (m, 6H), 1.37 (s, 9H), 1.28-0.82 (m, 3H).

Compound **57:** MS: m/z 848.2 (M⁺+1).

Compound **58:** MS: m/z 804.3 (M⁺+1); ¹H NMR (CDCl₃) δ 10.20 (s, 1H), 8.33 (d, 1H), 8.16 (dd, 1H), 7.77 (dd, 1H), 7.68-7.36 (m, 6H), 6.22 (s, 1H), 5.62 (q, 1H), 5.42 (d, 1H), 5.16 (dd, 1H), 4.99 (brs, 1H), 4.92 (dd, 1H), 4.68-4.60 (m, 1H), 4.37 (d, 1H), 4.16-4.08 (m, 1H), 3.73-3.35 (m, 4H), 3.08-2.87 (m, 2H), 2.69-2.40 (m, 2H), 2.32-0.81 (m, 17H).

Compound **59:** MS: m/z 804.3 (M⁺+1); ¹H NMR (CDCl₃) δ 10.18 (s, 1H), 8.34 (d, 1H), 8.17 (dd, 1H), 7.82 (dd, 1H), 7.73-7.40 (m, 4H), 7.35 (s, 1H), 6.23 (s, 1H), 5.63 (q, 1H), 5.48 (d, 1H), 5.17 (dd, 1H), 4.99 (brs, 1H), 4.91 (dd, 1H), 4.74-4.62 (m, 1H), 4.40 (d, 1H), 4.17-4.08 (m, 1H), 3.75-3.35 (m, 4H), 3.08-2.87 (m, 2H), 2.73-2.40 (m, 2H), 2.30-0.81 (m, 18H).

Compound **60:** MS: m/z 838.4 (M⁺+1).

### Example 59: Inhibition of NS3/4A Protease

### Protein expression and purification

A plasmid containing a gene encoding N-terminal His6-tagged-NS4A (21-32)-GSGS-NS3₍₃₋₁₈₁₎ was transformed into *E. coli* strain BL21(DE3) pLysS (Novagen) for protein over-expression. Single colony of transformed BL21 (DE3) pLysS was cultured in 200 mL of Lauria-Bertani (LB) medium with Kanamycin and Chloramphenicol at 37°C overnight. The bacterial culture was transferred into 6 L LB medium (Difco) containing antibiotics and incubated with shaking at 22°C. After the absorbance at 600 nm reached 0.6, the culture was induced with 1 mM isopropyl-1-thio-β-D-galactopyranoside (IPTG) at 22°C for 5 hours. The culture was subsequently harvested by centrifugation (6,000 xg for 15 minutes at 4°C). Cell pellets were resuspended in 150 mL buffer A (50 mM HEPES, pH 7.4, 0.3 M NaCl, 0.1% (w/v) CHAPS, 10 mM imidazol, 10% (v/v) glycerol). After the mixture was disrupted by four passes through a Microfluidizer operated at 30 psi, the cell debris was removed by centrifugation (58,250 x g for 30 minutes at 4°C). The cell lysate containing His₆-tagged proteins was charged at 3 mL/min onto a 25 mL Ni-NTA (Qiagen) column in the presence of 10 mM imidazole using a gradiFrac system (Pharmacia). The column was washed with 10 column volumes of the lysis buffer. The bound NS4A₍₂₁₋₃₂₎-GSGS-NS3₍₃₋₁₈₁₎ was eluted with 8 column volumes of buffer A supplemented with 300 mM imidazole. The pooled fractions were further purified by Q-Sepharose column equilibrated with buffer B (50 mM HEPES, pH 7.4, 0.1% (w/v) CHAPS, 10% (v/v) glycerol, 5 mM dithiothreitol (DTT), and 1 M NaCl). The eluant containing NS4A₍₂₁₋₃₂₎-GSGS-NS3₍₃₋₁₈₁₎ was collected and further purified by size-exclusion chromatography at a flow rate of 0.5 mL/min using the sephacryl-75 column (16 x 100 cm, Pharmacia) pre-equilibrated with buffer C (50 mM HEPES, pH 7.4, 0.1 % (w/v) CHAPS, 5 mM DTT, 10% (v/v) glycerol). The purified protein was frozen and stored at -80°C before use.

### HPLC Microbore assay

A solution containing 50 mM Tris, pH 7.4, 100 mM NaCl, 20% glycerol, 0.012% CHAPS, 10 mM DTT, 5 µM substrate Ac-Asp-Glu-Asp(EDANS)-Glu-Glu-Abu-ψ-[COOAla]-Ser-Lys(DABCYL)-NH₂ (RET S1, ANASPEC), and 10 µM test compound was prepared. 80 µL of the solution was added to each well of a 96-well plate. Reaction was initiated by addition of 20 µL of 10 nM NS3/4A protease in a buffer containing 50 mM Tris buffer, pH 7.4, 100 mM NaCl, 20% glycerol, and 0.012% CHAPS. The final concentration of NS3/4A protease was 2 nM, which was lower than the Km of substrate RET S 1.

The assay solution was incubated for 30 minutes at 30°C. The reaction was then terminated by addition of 100 µL of 1% TFA. 200 µL aliquot was transferred to each well of Agilent 96-well plates.

Reaction products were analyzed using reverse phase HPLC described below. The HPLC system included: Agilent 1100, Degasser G1379A, Binary pump G1312A, Autosampler G1367A, Column thermostated chamber G1316A, Diode array detector G1315B, Column: Agilent, ZORBAX Eclipse XDB-C18, 4.6 mm, 5 µm, P/N 993967-902, Column thermostat: room temperature, Injection volume: 100 µL, Solvent A = HPLC grade water + 0.09% TFA, Solvent B = HPLC grade acetonitrile + 0.09% TFA. Total HPLC running time was 7.6 minutes with a linear gradient from 25 to 50% solvent B in 4 minutes, 50% solvent B for 30 seconds, and a gradient from 50 to 25% solvent B for additional 30 seconds. The column was re-equilibrated with 25% solvent B for 2.6 minutes before next sample was injected. The IC₅₀ value (the concentration at which 50% inhibition of NS3/4A activity was observed) was calculated for each test compound based on the HPLC results.

Compounds 1-60 were tested in the above inhibition assay. The results showed that 54 compounds exhibited IC₅₀ values lower than 20 nM and 4 compounds exhibited IC₅₀ values in the range of 20-100 nM.

### Example 60: HCV Replicon Cell Assay Protocol

Cells containing HCV replicon were maintained in DMEM containing 10% fetal bovine serum (FBS), 1.0 mg/ml of G418, and appropriate supplements (media A).

On day 1, the replicon cell monolayer was treated with a trypsin/EDTA mixture, removed, and was diluted with media A to a final concentration of 48,000 cells/ml. The solution (1 ml) was added to each well of a 24-well tissue culture plate, and cultured overnight in a tissue culture incubator at 37°C with 5% CO₂.

On day 2, a test compound (in 100% DMSO) was serially diluted by DMEM containing 10% FBS and appropriate supplements (media B). The final concentration of DMSO was maintained at 0.2% throughout the dilution series.

The media on the replicon cell monolayer was removed, and then media B containing various concentrations of compounds was added. Media B without any compound was added to other wells as compound-free controls.

The cells were incubated with a compound or 0.2% DMSO in media B for 72 hours in a tissue culture incubator with 5% CO₂ at 37°C. Then, the media was removed and the replicon cell monolayer was washed once with PBS. RNA extraction reagents from RNeasy kits or TRIZOL reagents were added to the cells immediately to avoid degradation of RNA. Total RNA was extracted according to the instruction provided by manufacturer with modification to improve extraction efficiency and consistency. Finally, total cellular RNA, including HCV replicon RNA, was eluted and stored at -80°C until further processing.

A TaqMan® real-time RT-PCR quantification assay was set up with two sets of specific primers: one was for HCV and the other was for ACTB (beta-actin). The total RNA was added to the PCR reactions for quantification of both HCV and ACTB RNA in the same PCR well. Experimental failure was flagged and rejected based on the level of ACTB RNA in each well. The level of HCV RNA in each well was calculated according to a standard curve run in the same PCR plate. The percentage of inhibition of HCV RNA level by the compound treatment was calculated using the DMSO or compound-free control as 0% of inhibition. EC50 (concentration at which 50% inhibition of HCV RNA level was achieved) was calculated from the titration curve of any given compound.

Compounds 1-60 were tested in the HCV replicon cell assay. The results showed that 52 compounds exhibited EC₅₀ values lower than 20 nM and 3 compound exhibited EC₅₀ values in the range of 20-100 nM.

### OTHER EMBODIMENTS

All of the features disclosed in this specification may be combined in any combination. Each feature disclosed in this specification may be replaced by an alternative feature serving the same, equivalent, or similar purpose. Thus, unless expressly stated otherwise, each feature disclosed is only an example of a generic series of equivalent or similar features.

From the above description, one skilled in the art can easily ascertain the essential characteristics of the present invention, and without departing from the spirit and scope thereof, can make various changes and modifications of the invention to adapt it to various usages and conditions.

## Claims

1. A compound of formula (I): 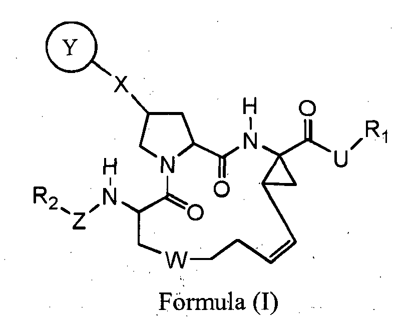 wherein
each of R₁ and R₂, independently, -is H, C₁₋₆ alkyl,C₃₋₁₀ cycloalkyl, C₁₋₁₀ heterocycloalkyl, aryl; or heteroaryl;
U is -O-, -NH-, -NH(CO)-, -NHSO-, or -NHSO₂-;
W is -(CH₂)ₘ-, -NH(CH₂)ₙ-, -(CH₂)ₙNH-, -O(CH₂)ₙ-, -(CH₂)ₙO-, -S(CH₂)ₙ-, -(CH₂)ₙS-, -SO-, -SO(CH₂)ₙ-, -(CH₂)ₙSO-, -SO₂(CH₂)ₙ-, or -(CH₂)ₙSO₂-, m being 1, 2, or 3, and n being 0, 1, or 2;
X is -O-;
Y is 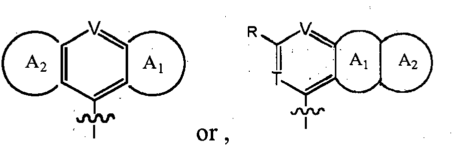 in which each of V and T, independently, is -CH- or -N-; R is H, halo, nitro, cyano, amino, C₁₋₆ alkyl, C₁₋₆ alkoxyl, C₂₋₆ alkenyl, C₂₋₆ alkynyl, C₃₋₁₀ cycloalkyl, C₁₋₁₀ heterocycloalkyl, aryl, or heteroaryl; and each of A₁ and A₂, independently, is C₄₋₁₀ cycloalkyl, C₁₋₁₀ heterocycloalkyl, aryl, or heteroaryl, each of which is optionally substituted with halo, nitro, cyano, amino,
C₁₋₆ alkyl, C₁₋₆ alkoxyl, C₂₋₆ alkenyl, C₂₋₆ alkynyl, aryl, or heteroaryl; or optionally fused with another C₃₋₁₀ cycloalkyl, C₁₋₁₀ heterocycloalkyl, aryl, or heteroaryl, each of which is optionally substituted with halo, nitro, cyano, C₁₋₆ alkyl, C₁₋₆ alkoxyl, C₂₋₆ alkenyl, C₂₋₆ alkynyl, C₃₋₁₀ cycloalkyl, C₁₋₁₀ heterocycloalkyl, aryl, or heteroaryl; and
Z is -C(O), -OC(O)-, -NR'C(O)-, -OC(S)-, -NR'-C(S)-, or -OC(NH)-; in which R' is H, C₁₋₆ alkyl, C₃₋₁₀ cycloalkyl, C₁₋₁₀ heterocycloalkyl, aryl, or heteroaryl.

2. The compound of claim 1, wherein W is -CH₂CH₂-, -OCH₂-, -SCH₂-, or -SOCH₂-.

3. The compound of claim 1, wherein Y is 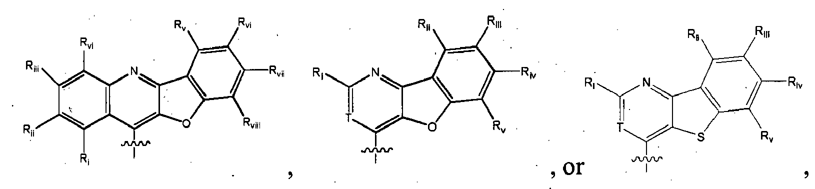 wherein each of Rᵢ, Rᵢᵢ, Rᵢᵢᵢ, Rᵢᵥ, Rᵥ, Rᵥᵢ, Rᵥᵢᵢ, and Rᵥᵢᵢᵢ is, independently, H, halo, nitro, cyano, amino, C₁₋₆ alkyl, C₁₋₆ alkoxyl, amino, C₂₋₆ alkenyl, C₂₋₆ alkynyl, C₃₋₁₀ cycloalkyl, C₁₋₁₀ heterocycloalkyl, aryl, or heteroaryl; and T is defined in claim 1.

4. The compound of claim 3, wherein W is -CH₂CH₂-, -OCH₂-, -SCH₂-, or -SOCH₂-, X is O, Z is -OC(O)- and wherein U is optionally -NHSO₂-.

5. The compound of claim 4, wherein U is -NHSO₂-, R₁ is cyclopropyl, and R₂ is optionally C₁₋₅ alkyl or C₃₋₈ cycloalkyl.

6. The compound of claim 1, wherein X is O, Z is -OC(O)-, U is -NHSO₂-, R₁ is cyclopropyl, and R₂ is C₁₋₅ alkyl or C₃₋₈ cycloalkyl.

7. The compound of claim 1, wherein the compound has the stereochemistry as shown below:

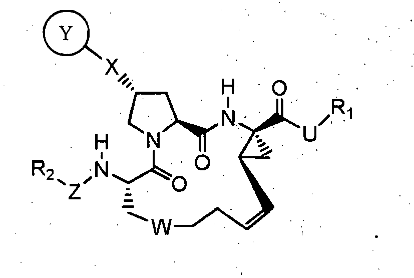

8. The compound of claim 1, wherein the compound is one of the following compounds:

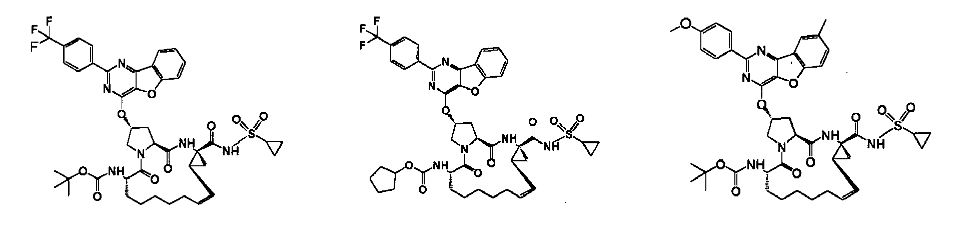

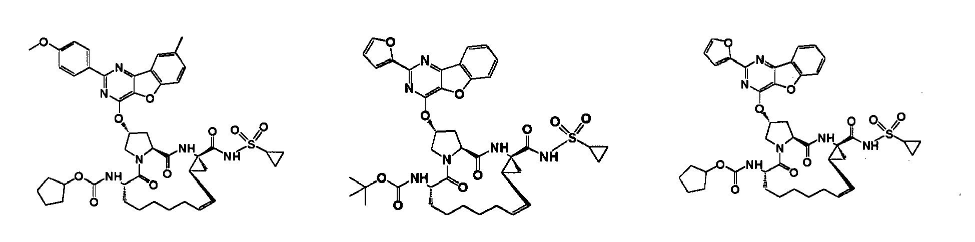

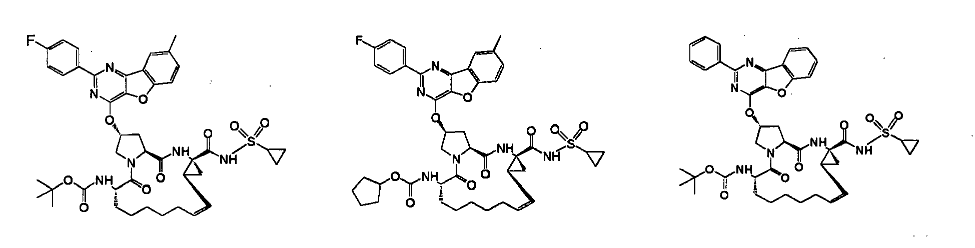

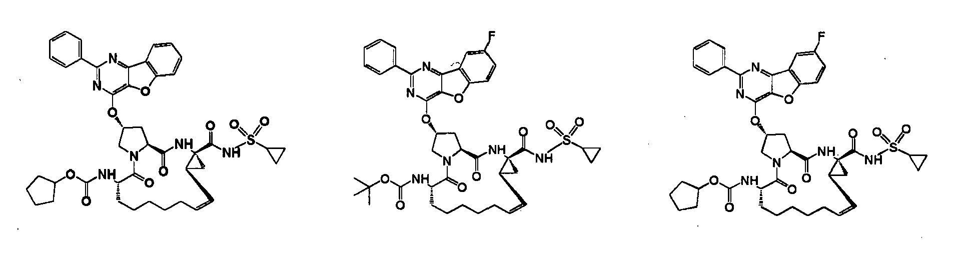

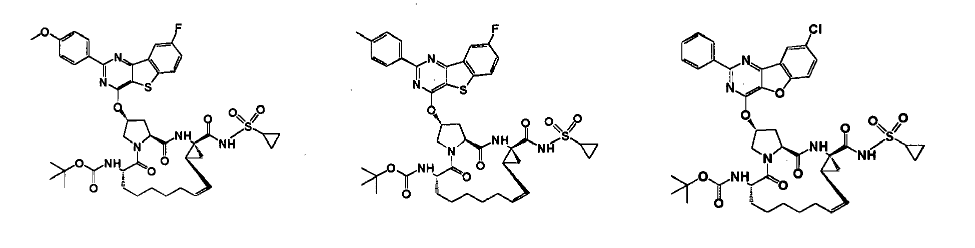

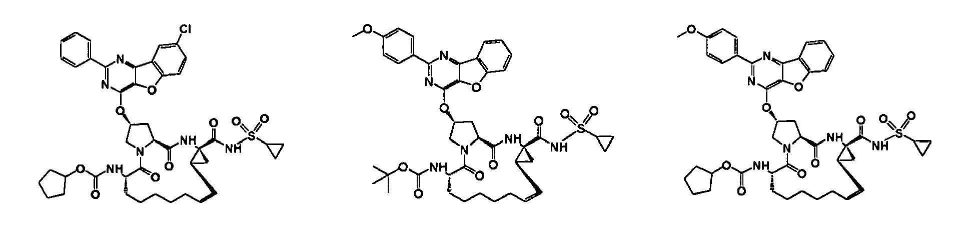

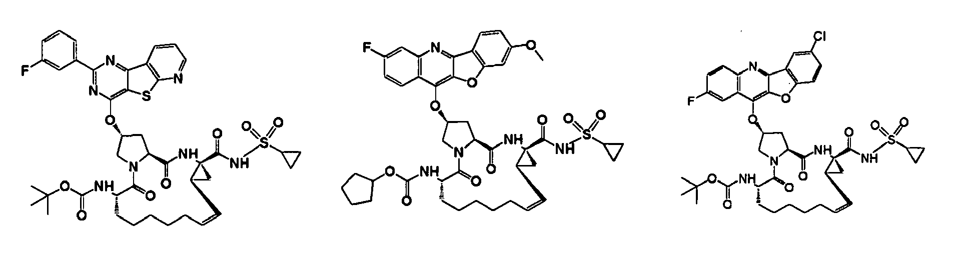

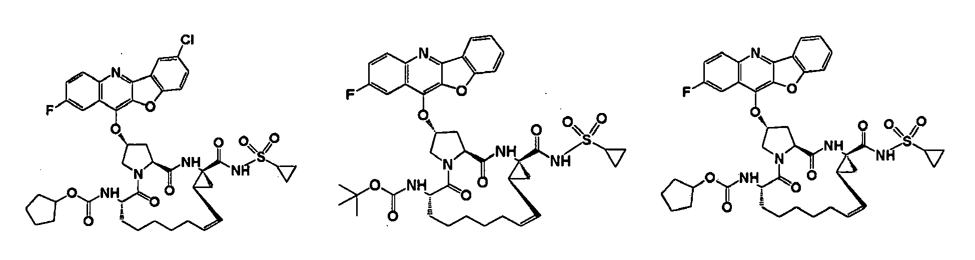

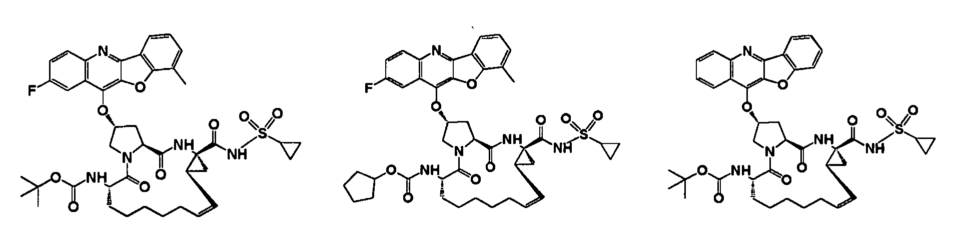

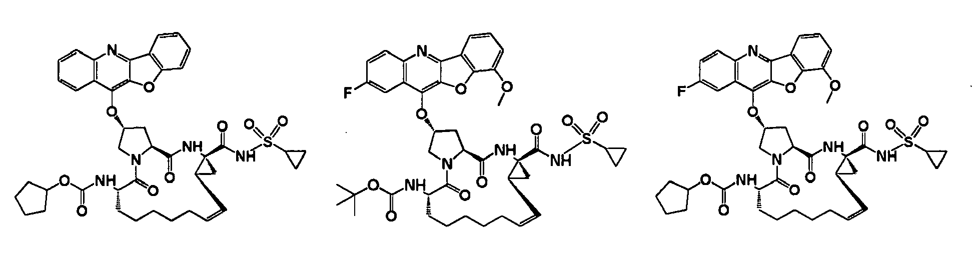

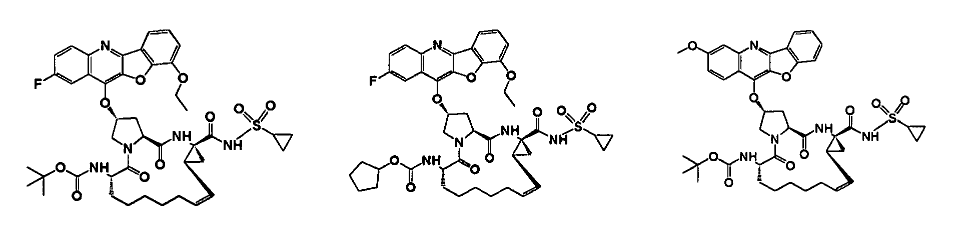

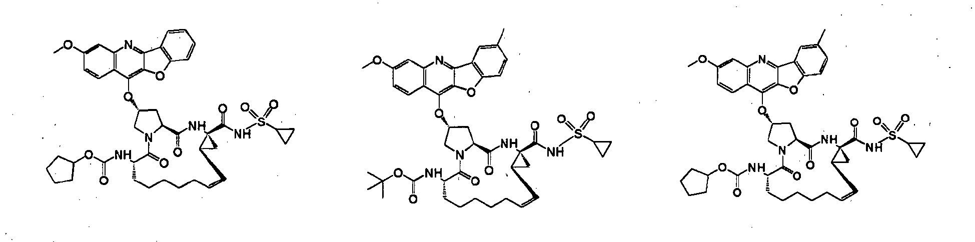

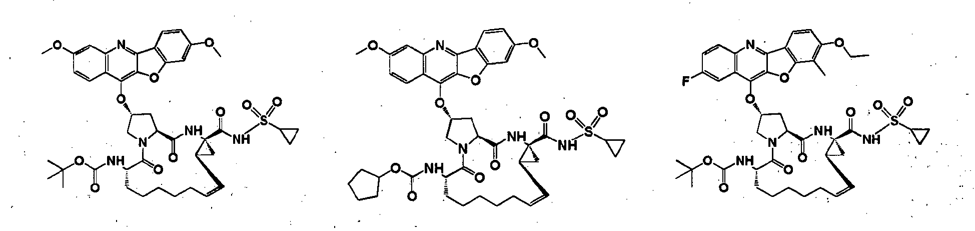

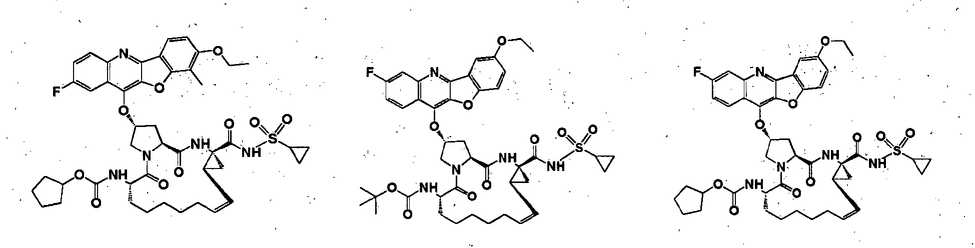

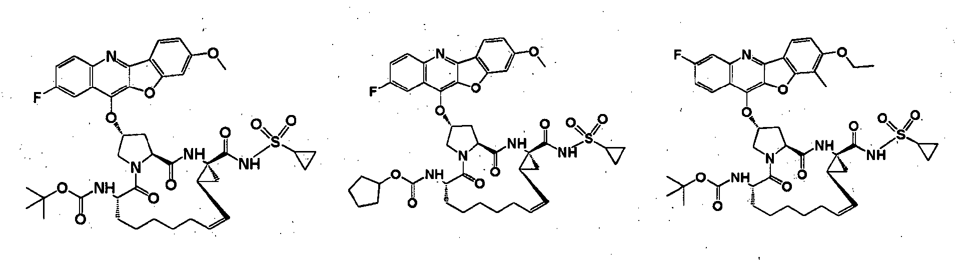

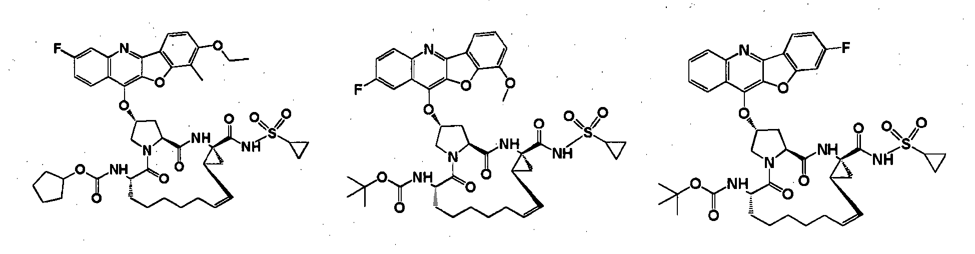

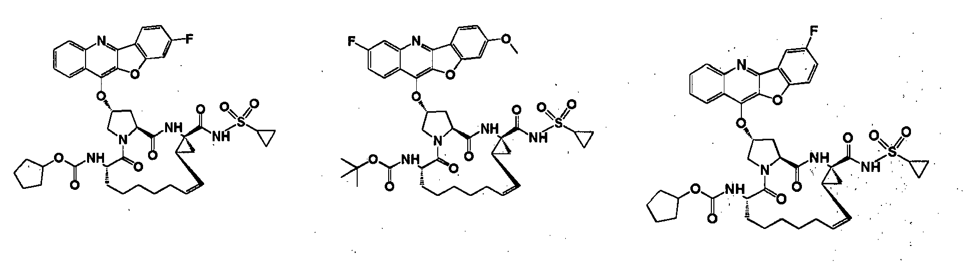

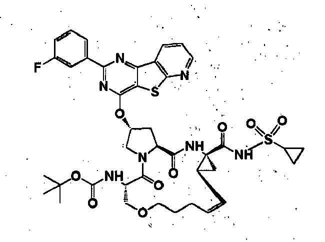

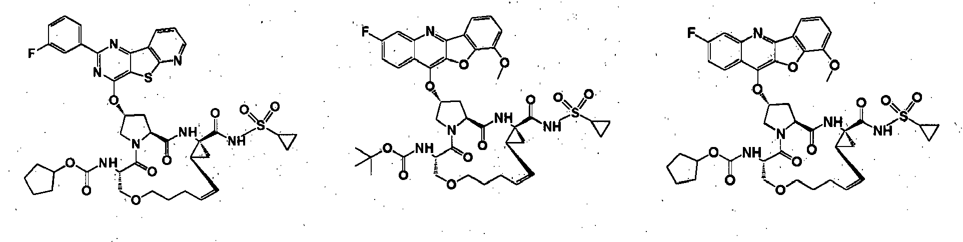

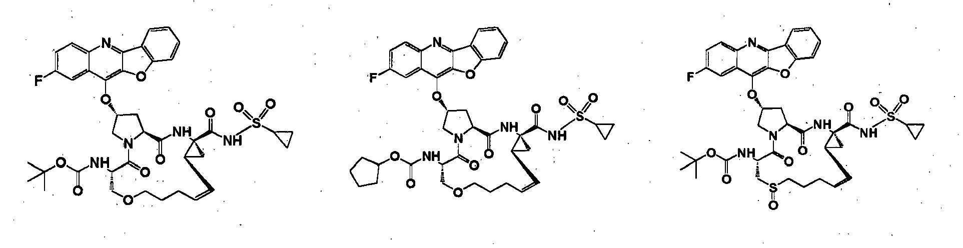

9. A pharmaceutical composition comprising an antiviral agent having the formula recited in claim 1 and a pharmaceutically acceptable carrier, and optionally further comprising an immunomodulatory agent, another antiviral agent, or an inhibitor of NS5B polymerase, NS5A, NS4B, or p7.

10. A compound of any of claims 1 or 8, for use in treating hepatitis C virus infection.

## Patentansprüche

1. Eine Verbindung nach Formel (I): 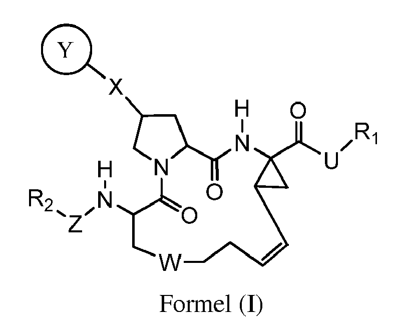 wobei
jeweils R₁ und R₂ unabhängig H, C₁₋₆ Alkyl, C₃₋₁₀ Cycloalkyl, C₁₋₁₀ Heterocycloalkyl, Aryl oder Heteroaryl ist;
U -O-, -NH-, -NH(CO)-, -NHSO- oder -NHSO₂- ist;
W -(CH₂)ₘ-, -NH(CH₂)ₙ-, -(CH₂)ₙNH-, -O(CH₂)ₙ₋, -(CH₂)ₙO-, -S(CH₂)ₙ-, -(CH₂)ₙS-, -SO-, -SO(CH₂)ₙ-, -(CH₂)ₙSO-, -SO₂(CH₂)ₙ- oder -(CH₂)ₙSO₂- ist, m 1, 2 oder 3 ist und n 0, 1 oder 2 ist;
X -O- ist;
Y 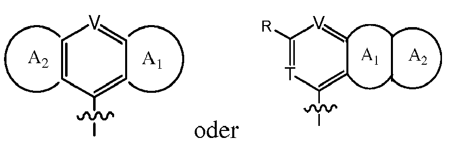 ist, wobei jeweils V und T unabhängig -CH- oder -N- ist; R H, Halo, Nitro, Cyano, Amino, C₁₋₆ Alkyl, C₁₋₆ Alkoxyl, C₂₋₆ Alkenyl, C₂₋₆ Alkinyl, C₃₋₁₀ Cycloalkyl, C₁₋₁₀ Heterocycloalkyl, Aryl oder Heteroaryl ist; und jeweils A₁ und A₂ unabhängig C₄₋₁₀ Cycloalkyl, C₁₋₁₀ Heterocycloalkyl, Aryl oder Heteroaryl ist, jedes von diesen ist optional substituiert mit Halo, Nitro, Cyano, Amino, C₁₋₆ Alkyl, C₁₋₆ Alkoxyl, C₂₋₆ Alkenyl, C₂₋₆ Alkinyl, Aryl oder Heteroaryl; oder optional fusioniert mit einem anderen C₃₋₁₀ Cycloalkyl, C₁₋₁₀ Heterocycloalkyl, Aryl oder Heteroaryl, jedes von diesen ist optional substituiert mit Halo, Nitro, Cyano, C₁₋₆ Alkyl, C₁₋₆ Alkoxyl, C₂₋₆ Alkenyl, C₂₋₆ Alkinyl, C₃₋₁₀ Cycloalkyl, C₁₋₁₀ Heterocycloalkyl, Aryl oder Heteroaryl; und
Z -C(O), -OC(O)-, -NR'C(O)-, -OC(S)-, -NR'-C(S)- oder -OC(NH)- ist; wobei R' H, C₁₋₆ Alkyl, C₃₋₁₀ Cycloalkyl, C₁₋₁₀ Heterocycloalkyl, Aryl oder Heteroaryl ist.

2. Die Verbindung nach Anspruch 1, wobei W -CH₂CH₂-, -OCH₂-, -SCH₂- oder -SOCH₂- ist.

3. Die Verbindung nach Anspruch 1, wobei Y 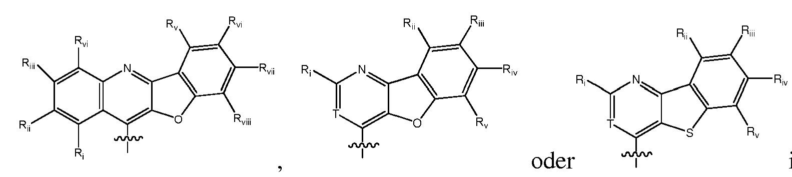 ist, wobei jeweils Rᵢ, Rᵢᵢ, Rᵢᵢᵢ, Rᵢᵥ, Rᵥ, Rᵥᵢ, Rᵥᵢᵢ und Rᵥᵢᵢᵢ unabhängig H, Halo, Nitro, Cyano, Amino, C₁₋₆ Alkyl, C₁₋₆ Alkoxyl, Amino, C₂₋₆ Alkenyl, C₂₋₆ Alkinyl, C₃₋₁₀ Cycloalkyl, C₁₋₁₀ Heterocycloalkyl, Aryl oder Heteroaryl ist; und T wie in Anspruch 1 definiert ist.

4. Die Verbindung nach Anspruch 3, wobei W -CH₂CH₂-, -OCH₂-, -SCH₂- oder -SOCH₂- ist, X O ist, Z -OC(O)- ist und wobei U optional -NHSO₂- ist.

5. Die Verbindung nach Anspruch 4, wobei U -NHSO₂- ist, R₁ Cyclopropyl ist und R₂ optional C₁₋₅ Alkyl oder C₃₋₈ Cycloalkyl ist.

6. Die Verbindung nach Anspruch 1, wobei X O ist, Z -OC(O)- ist, U -NHSO₂- ist, R₁ Cyclopropyl ist und R₂ C₁₋₅ Alkyl oder C₃₋₈ Cycloalkyl ist.

7. Die Verbindung nach Anspruch 1, wobei die Verbindung die Stereochemie aufweist wie unten gezeigt:

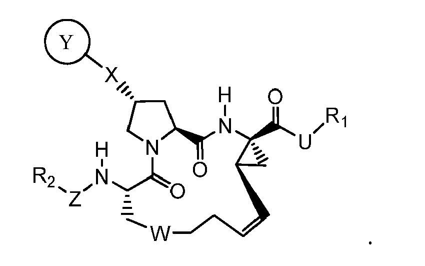

8. Die Verbindung nach Anspruch 1, wobei die Verbindung eine der folgenden Verbindungen ist:

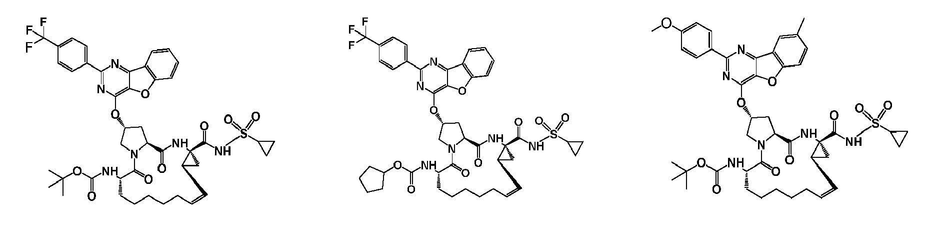

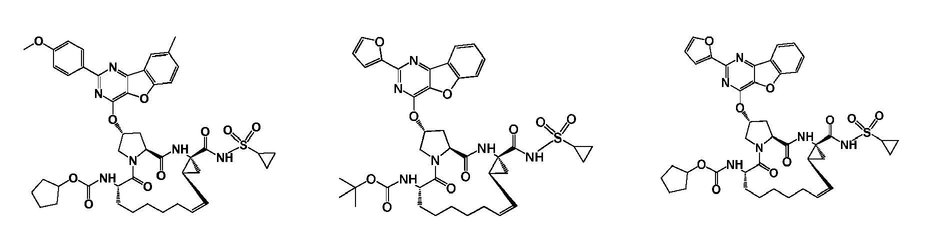

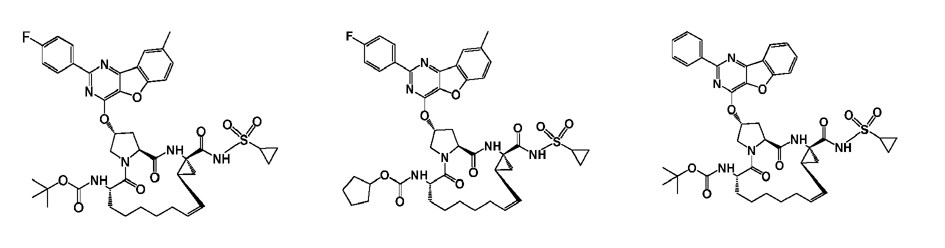

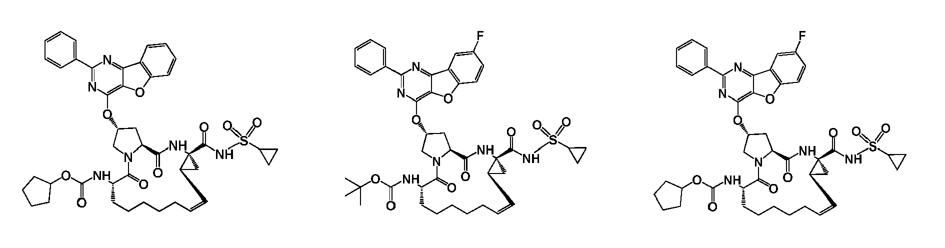

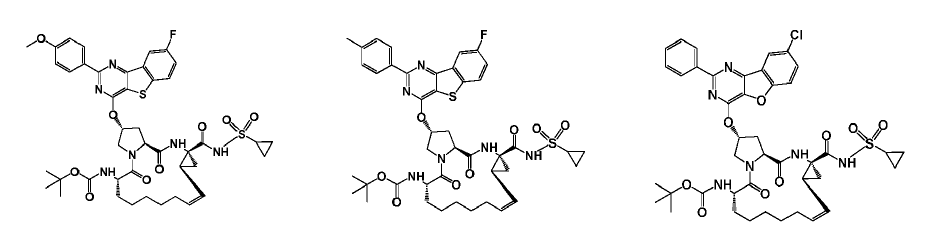

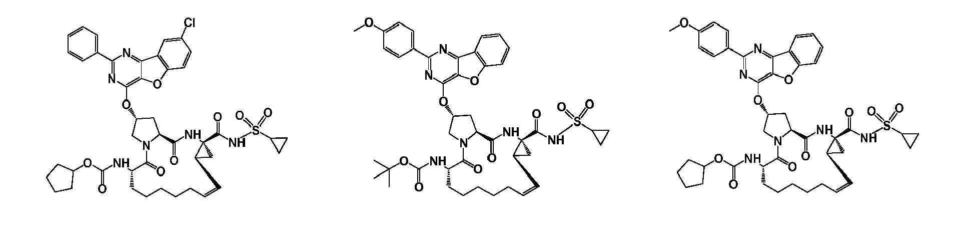

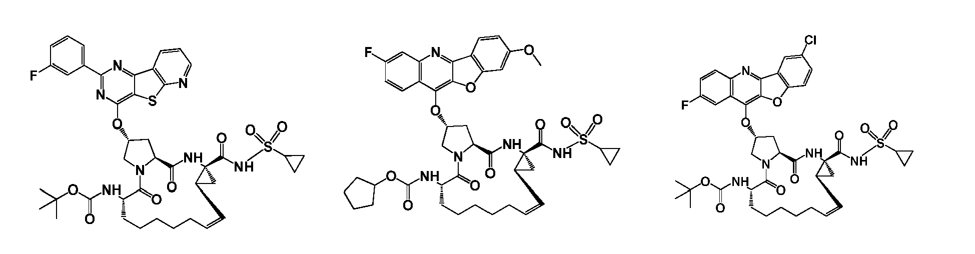

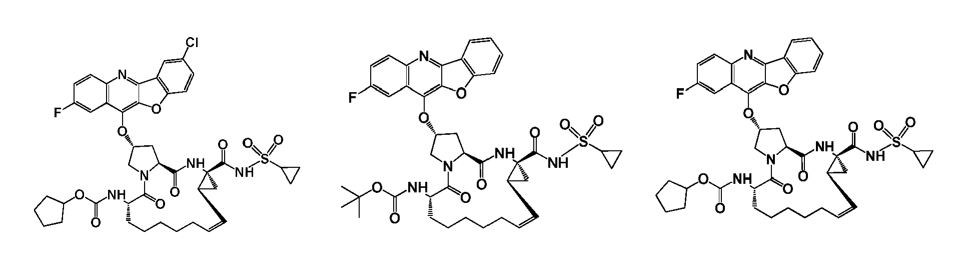

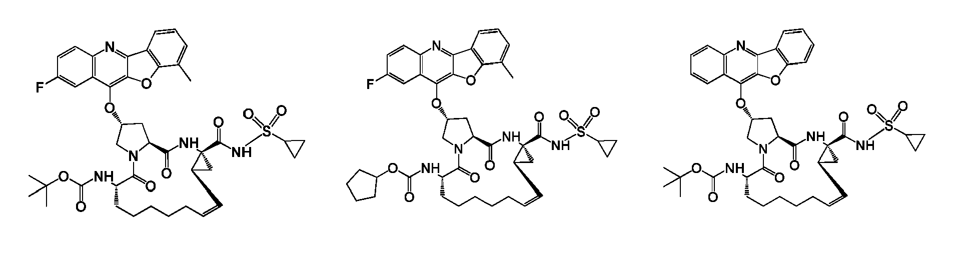

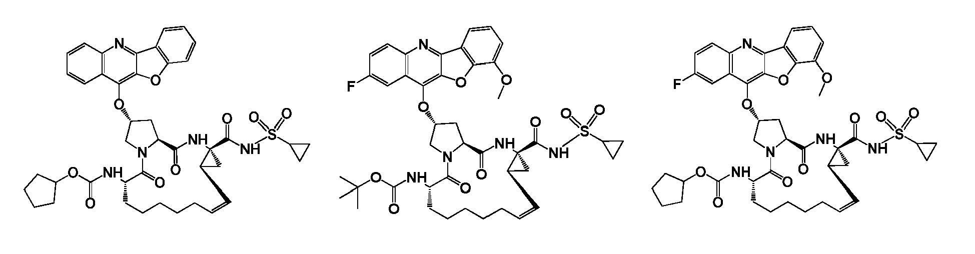

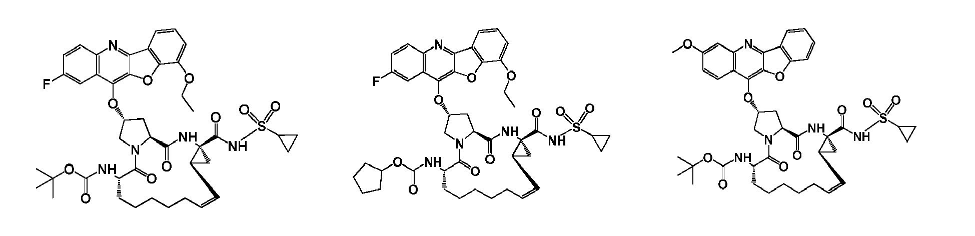

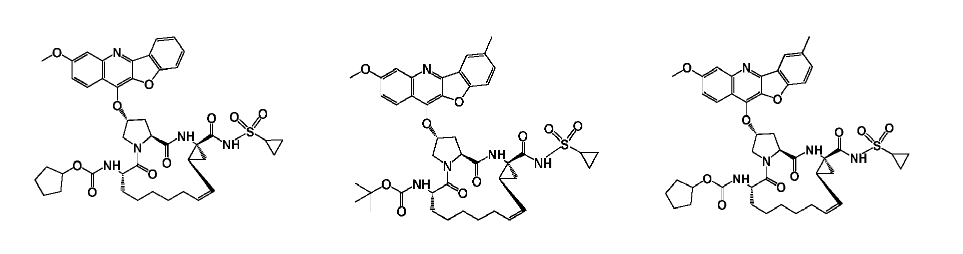

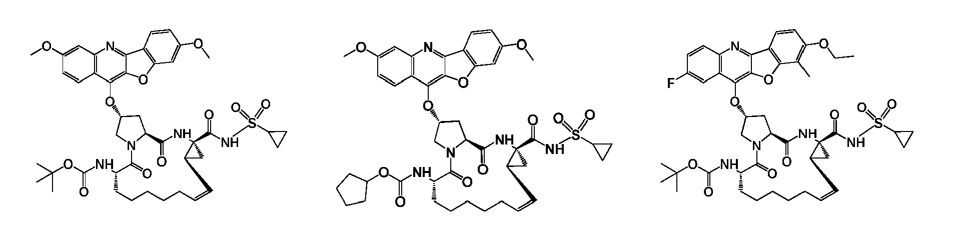

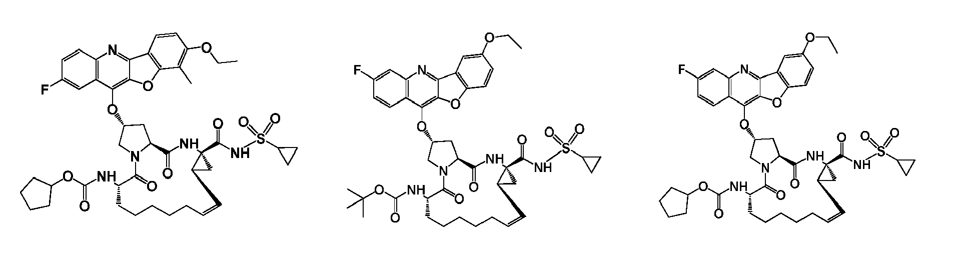

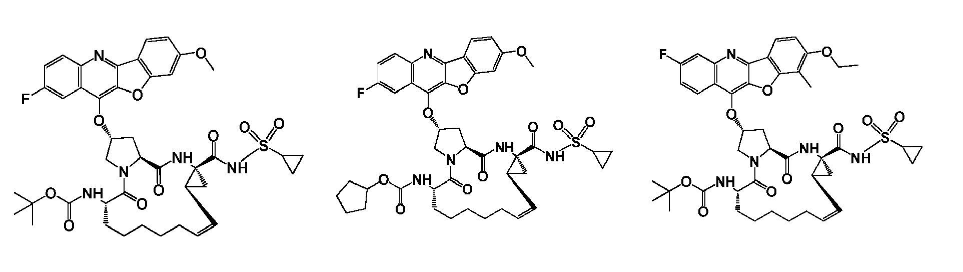

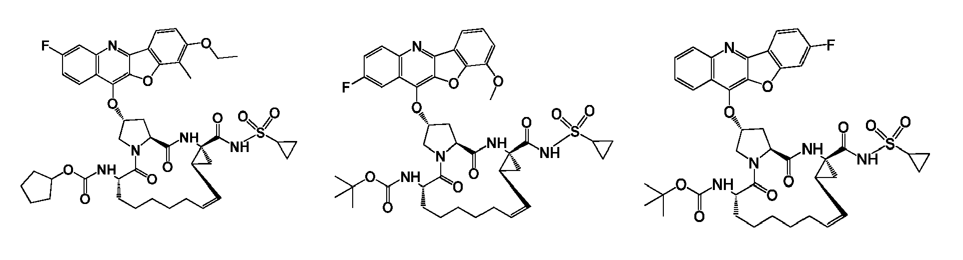

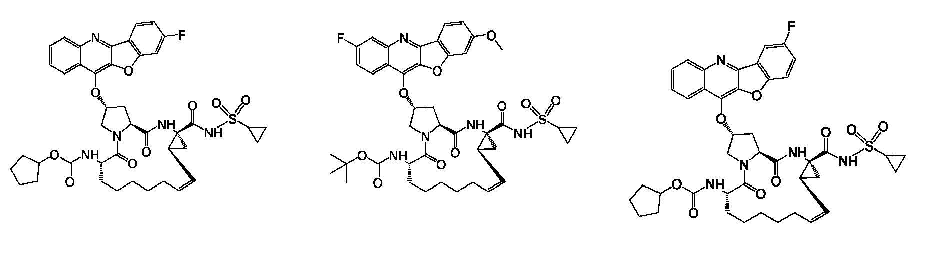

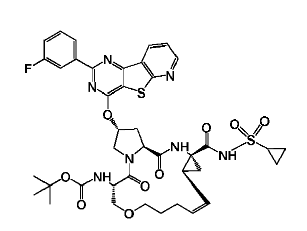

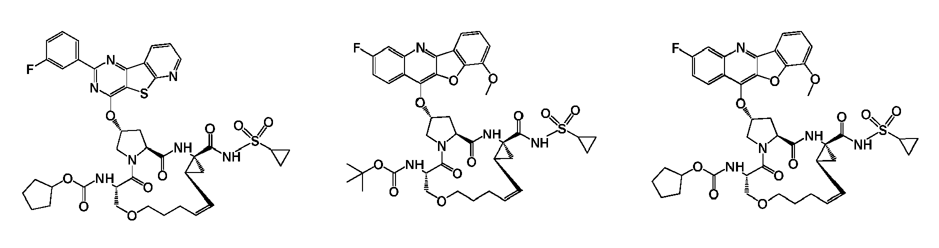

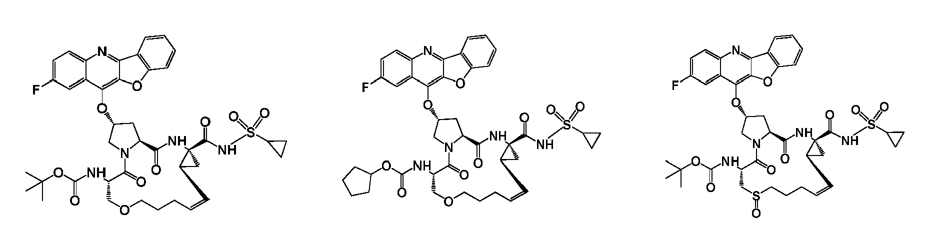

9. Eine pharmazeutische Zusammensetzung umfassend einen antiviralen Wirkstoff, der die Formel wie in Anspruch 1 vorgetragen aufweist, und einen pharmazeutisch verträglichen Träger, und optional ferner umfassend einen immunregulierenden Wirkstoff, einen anderen antiviralen Wirkstoff oder einen Inhibitor der Polymerase NS5B, NS5A, NS4B, oder p7.

10. Eine Verbindung nach einem der Ansprüche 1 oder 8 zur Verwendung bei der Behandlung einer Infektion mit dem Hepatitis-C-Virus.

## Revendications

1. Composé de formule (I): 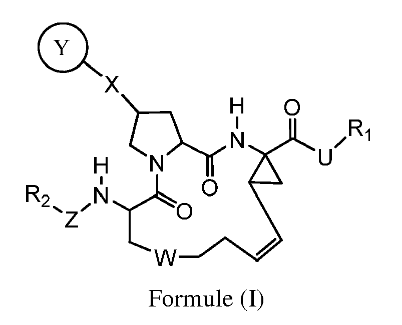 dans laquelle
chaque R1 et R2 est indépendamment H, alkyle en C₁₋₆, cycloalkyle en C₃₋₁₀, hétérocycloalkyle en C₁₋₁₀, aryle, ou hétéroaryle ;
U est -O-, -NH-, -NH(CO)-, -NHSO-, ou -NHSO₂-;
W est -(CH₂)ₘ-, -NH(CH₂)ₙ-, -(CH₂)ₙNH-, -O(CH₂)ₙ-, -(CH₂)ₙO-, -S(CH₂)ₙ-, -(CH₂)ₙS-, -SO-, -SO(CH₂)ₙ-, -(CH₂)ₙSO-, -SO₂(CH₂)ₙ-, ou -(CH₂)ₙSO₂-, m étant 1, 2, ou 3 et n étant 0, 1, ou 2;
X est -O-;
Y est 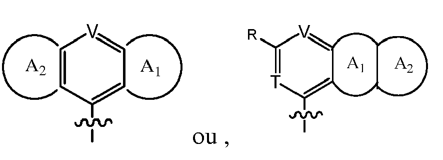 dans lequel chaque V et T est indépendamment -CH- ou -N-; R est H, halogéno, nitro, cyano, amino, alkyle en C₁₋₆, alcoxyle en C₁₋₆, alcényle en C₂₋₆, alcynyle en C₂₋₆, cycloalkyle en C₃₋₁₀, hétérocycloalkyle en C₁₋₁₀, aryle, ou hétéroaryle; et chaque A₁ et A₂ est indépendamment cycloalkyle en C₄₋₁₀, hétérocycloalkyle en C₁₋₁₀, aryle, ou hétéroaryle, dont chacun est de façon optionnelle substitué avec halogéno, nitro, cyano, amino, alkyle en C₁₋₆, alcoxyle en C₁₋₆, alcényle en C₂₋₆, alcynyle en C₂₋₆, aryle, ou hétéroaryle; ou de façon optionnelle fusionné avec un autre cycloalkyle en C₃₋₁₀, hétérocycloalkyle en C₁₋₁₀, aryle, ou hétéroaryle, dont chacun est de façon optionnelle substitué avec halogéno, nitro, cyano, alkyle en C₁₋₆, alcoxyle en C₁₋₆, alcényle en C₂₋₆, alcynyle en C₂₋₆, cycloalkyle en C₃₋₁₀, hétérocycloalkyle en C₁₋₁₀, aryle, ou hétéroaryle ; et
Z est -C(O), -OC(O)-, -NR'C(O)-, -OC(S)-, -NR'-C(S)-, ou -OC(NH)-; dans lequel R' est H, alkyle en C₁₋₆, cycloalkyle en C₃₋₁₀, hétérocycloalkyle en C₁₋₁₀, aryle, ou hétéroaryle.

2. Composé selon la revendication 1, dans lequel W est -CH₂CH₂-, -OCH₂-, -SCH₂-, ou -SOCH₂-.

3. Composé selon la revendication 1, dans lequel Y est 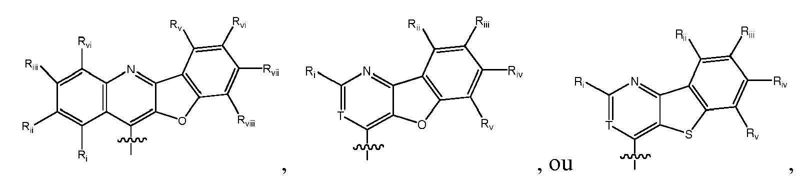 dans lequel chaque Rᵢ, Rᵢᵢ, Rᵢᵢᵢ, Rᵢᵥ, Rᵥ, Rᵥᵢ, Rᵥᵢᵢ, et Rᵥᵢᵢᵢ est indépendamment H, halo, nitro, cyano, amino, alkyle en C₁₋₆, alcoxyle en C₁₋₆, amino, alcényle en C₂₋₆, alcynyle en C₂₋₆, cycloalkyle en C₃₋₁₀, hétérocycloalkyle en C₁₋₁₀, aryle, ou hétéroaryle; et T est défini dans la revendication 1.

4. Composé selon la revendication 3 dans lequel W est -CH₂CH₂-, -OCH₂-, -SCH₂-, ou -SOCH₂-, X est O, Z est -OC(O)- et dans lequel U est de façon optionnelle -NHSO₂-.

5. Composé selon la revendication 4, dans lequel U est -NHSO₂-, R₁ est un cyclopropyle, et R₂ est de façon optionnelle un alkyle en C₁₋₅ ou un cycloalkyle en C₃₋₈.

6. Composé selon la revendication 1, dans lequel X est O, Z est -OC(O)-, U est - NHSO₂-, R₁ est un cyclopropyle, et R₂ est un alkyle en C₁₋₅ ou un cycloalkyle en C₃₋₈.

7. Composé selon la revendication 1, dans lequel le composé possède la stéréochimie comme montrée ci dessous:

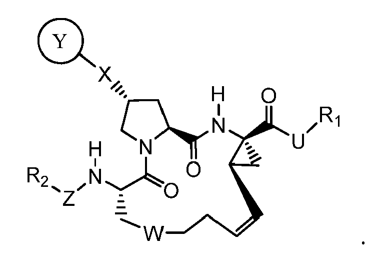

8. Composé selon la revendication 1, dans lequel le composé est un des composés suivants:

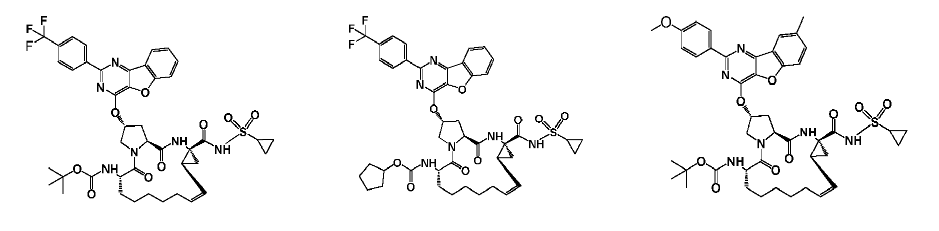

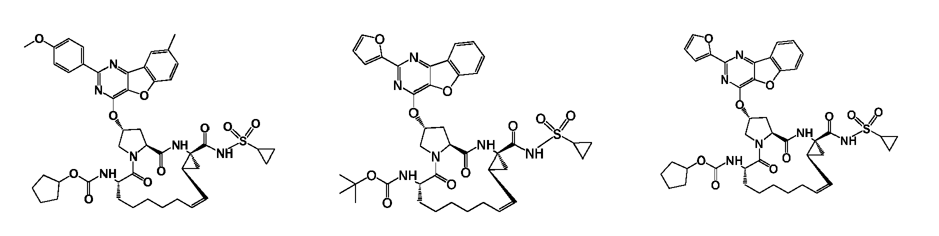

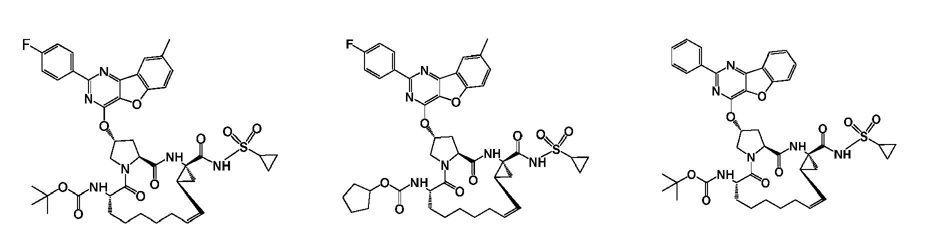

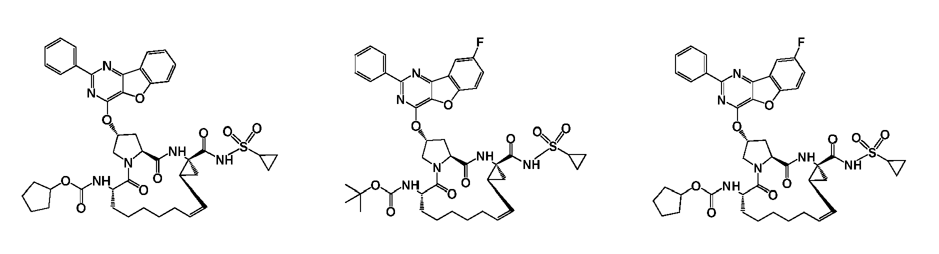

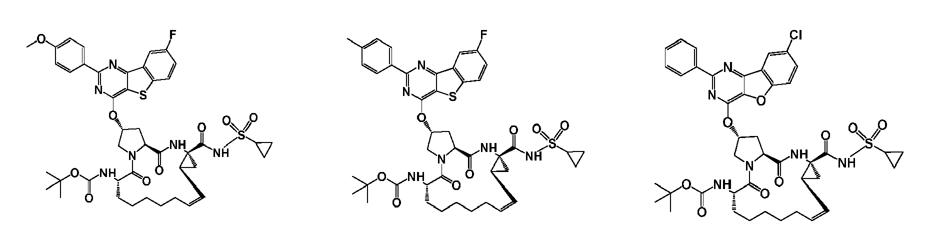

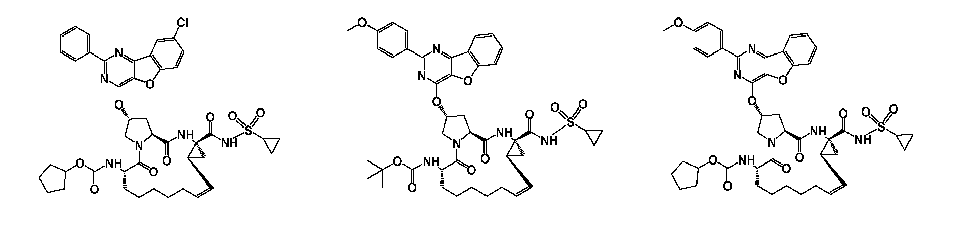

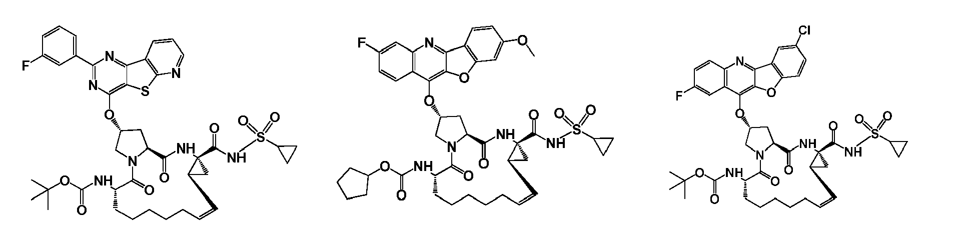

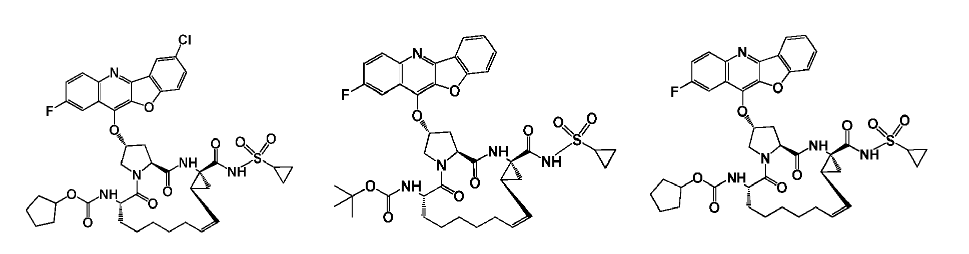

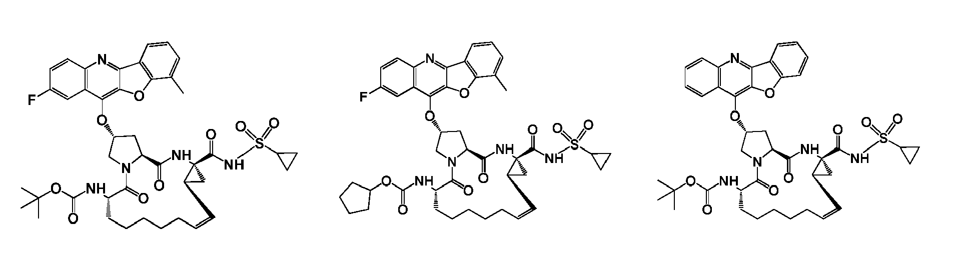

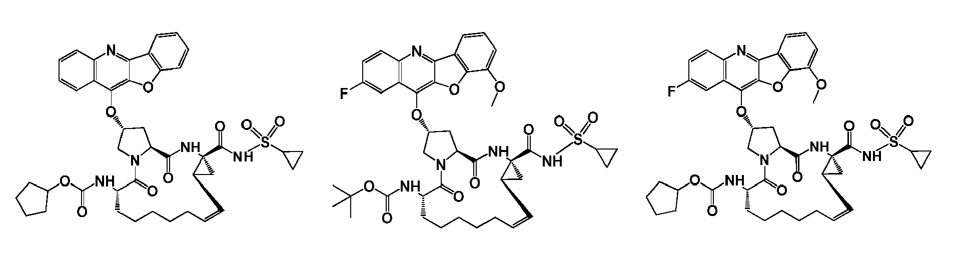

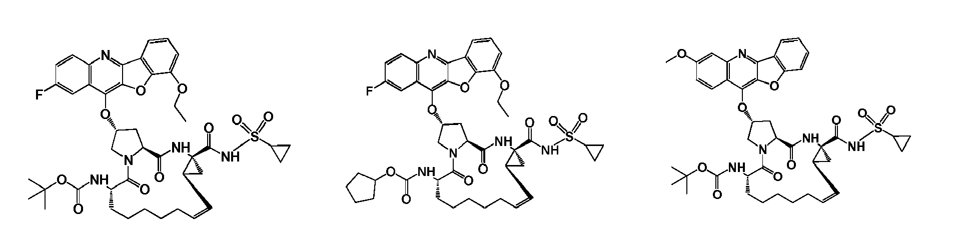

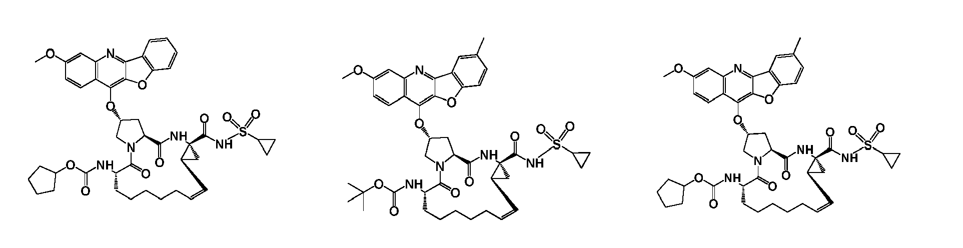

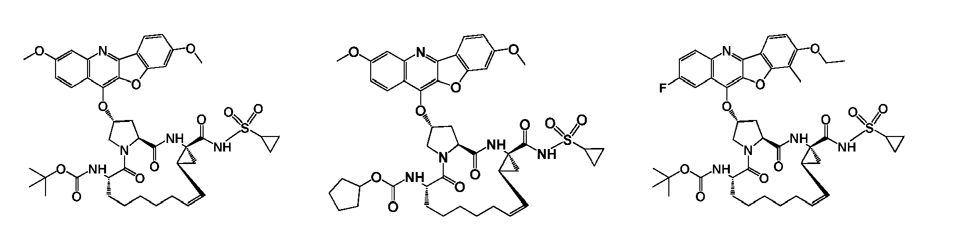

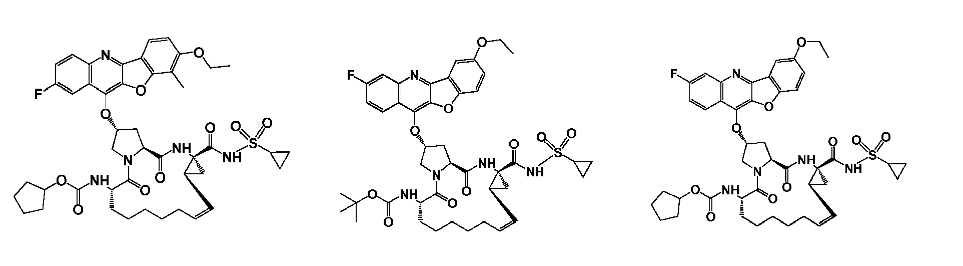

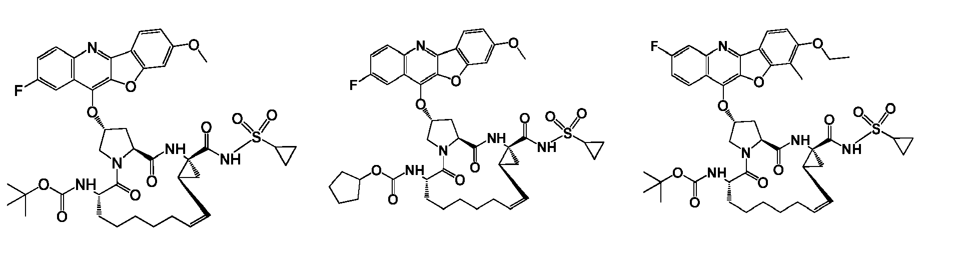

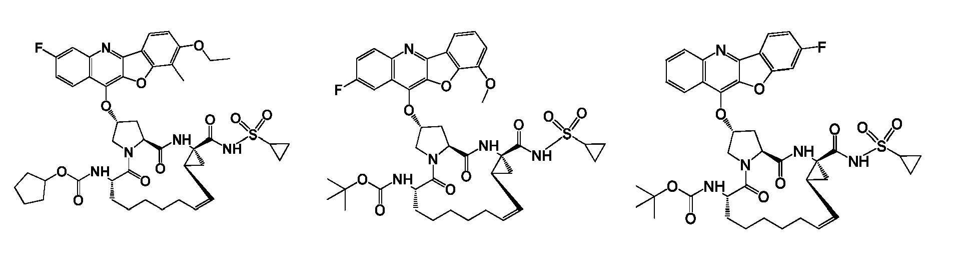

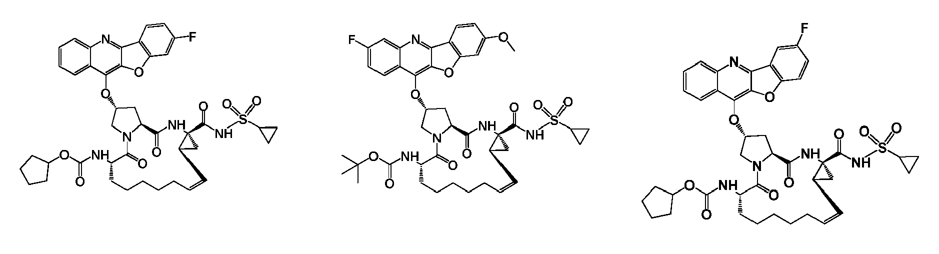

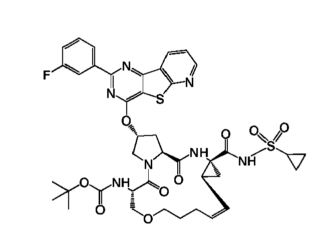

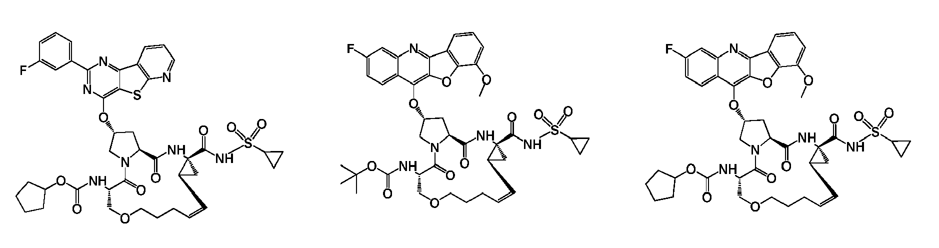

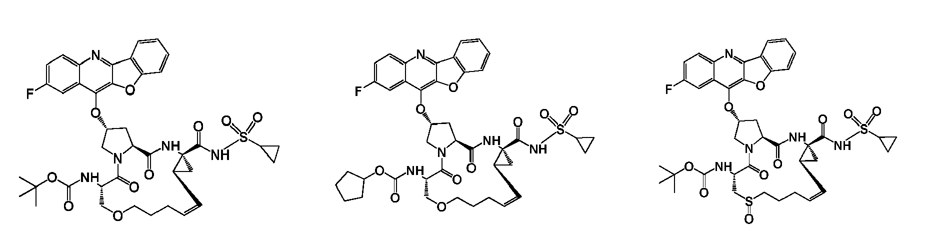

9. Composition pharmaceutique comprenant un agent antiviral ayant la formule selon la revendication 1 et un véhicule pharmaceutiquement acceptable, et comprend en outre, de façon optionnelle, un agent immunomodulateur, un autre agent antiviral, ou un inhibiteur de la polymérase NS5B, NS5A, NS4B ou p7.

10. Composé selon l'une quelconque des revendications 1 ou 8, pour son utilisation dans le traitement d'une infection au virus de l'hépatite C.
